# EUROPEAN PATENT SPECIFICATION

(11) **EP 1 399 156 B1**
(45) Date of publication and mention of the grant of the patent: **22.11.2006**
(21) Application number: 02735418.2
(22) Date of filing: 14.06.2002
(51) Int. Cl.: A61K 31/415, A61P 3/10, A61P 15/08, C07D 405/06

(54) **ALKYLIDENE PIRAZOLIDINEDIONE DERIVATIVES AND THEIR USE FOR TREATING DIABETES AND OBESITY**
ALKYLIDENE PIRAZOLIDINEDIONE DERIVATE UND DEREN VERWENDUNG GEGEN DIABETES UND DIABESITAS
DERIVES D'ALKYLIDENE PYRAZOLIDINEDIONE ET LEURS UTILISATION DANS LE TRAITEMENT DU DIABETE ET DE L'OBESITE

(30) Priority: 18.06.2001 EP 01113633
(43) Date of publication of application: 24.03.2004
(73) Proprietor: Applied Research Systems ARS Holding N.V., Curacao (AN)
(72) Inventor: BOMBRUN, Agnès, F-74560 Monnetier-Mornex (FR); RUECKLE, Thomas, 1228 Plan-les-Ouates (CH); SWINNEN, Dominique, CH-1212 Grand-Lancy (CH); GONZALEZ, Jérôme, F-74100 Annemasse (FR); CHURCH, Dennis, CH-1291 COMMUGNY (CH)
(74) Representative: Hassa, Jürgen
(86) International application number: PCT/EP2002/006627
(87) International publication number: WO 2002/102359

(56) References cited:
- EP-A- 0 028 410
- WO-A-01/00585
- US-A- 4 591 594
- US-B1- 6 211 221
- DATABASE CHEMCATS [Online] chemical abstract service, columbus, ohio, us; XP002195423 & "scientific exchange product list" 1 June 2001 (2001-06-01) , SCIENTIFIC EXCHANGE INC. , NEWARK USA & "Ambinter: exploratory library" 31 May 2001 (2001-05-31) , AMBINTER , PARIS, FRANCE & "Timtec stock screening collection" 19 February 2001 (2001-02-19) , TIMTEC CORPORATION & "AsinEx compound collection" 10 May 2001 (2001-05-10) , ASINEX , MOSCOW RUSSIA
- BUZZETTI, FRANCO ET AL: "Cinnamamide analogs as inhibitors of protein tyrosine kinases" FARMACO (1993), 48(5), 615-36, XP002103536
- KHALIL, ZARIF HALEEM ET AL: "Synthesis and application of some 1-[8-hydroxy-5(or 7)- quinolinyl]alkylidene-substituted heterocycles as bactericides, fungicides, and bioregulators" BULL. CHEM. SOC. JPN. (1988), 61(4), 1345-9, XP000990042
- SHIRAISHI, TADAYOSHI ET AL: "Specific inhibitors of tyrosine-specific protein kinases: properties of 4-hydroxycinnamamide derivatives in vitro" CANCER RES. (1989), 49(9), 2374-8, XP008002000
- "Merck Manual", section 2, chapter "Diabetes mellitus"" MERCK RESEARCH LABORATORIES , USA
- ESPANEL: "Pulling strings below..." ENDOCRINE, vol. 15, no. 1, 2001, pages 19-28,

## Description

### Field of the invention

The present invention is related to the use of alkylidene pyrazolidinedione derivatives of formula (I) for the treatment and/or prevention of metabolic disorders related to insulin resistance or hyperglycemia, namely obesity type II diabetes, type I diabetes. The present invention is furthermore related to novel alkylidene pyrazolidinedione derivatives and a method of preparing them.

### Background of the invention

The prevalence of insulin resistance in glucose intolerant subjects is well known. Reaven et al (*American Journal of Medicine* 1976, 60, 80) used a continuous infusion of glucose and insulin (insulin/glucose clamp technique) and oral glucose tolerance tests to demonstrate that insulin resistance exists in a diverse group of non-obese, non-ketotic subjects. These subjects ranged from borderline glucose tolerant to overt, fasting hyperglycemia. The diabetic groups in these studies included both insulin dependent (IDDM) and non-insulin dependent (NIDDM) subjects.

Coincident with sustained insulin resistance is the more easily determined hyperinsulinemia, which may be measured by accurate determination of circulating plasma insulin concentration in the plasma of subjects. Hyperinsulinemia may be present as a result of insulin resistance, such as is in obese and/or diabetic (NIDDM) subjects and/or glucose intolerant subjects, or in IDDM subjects, as a consequence of over injection of insulin compared with normal physiological release of the hormone by the endocrine pancreas.

The association of hyperinsulinemia and insulin resistance with obesity and with ischemic diseases of the large blood vessels (e.g. atherosclerosis) has been well established by numerous experimental, clinical and epidemiological studies (Stout, *Metabolism* 1985, 34, 7). Statistically significant plasma insulin elevations at 1 and 2 hours after oral glucose load correlate with an increased risk of coronary heart disease.

Since most of these studies actually excluded diabetic subjects, data relating the risk of atherosclerotic diseases to the diabetic condition are not as numerous, but point in the same direction as for non-diabetic subjects. However, the incidence of atherosclerotic diseases in morbidity and mortality statistics in the diabetic population exceeds that of the nondiabetic population (Pyorala et al; Jarrett *Diabetes*/*Metabolism Reviews* 1989, 5, 547).

The association of hyperinsulinemia and insulin resistance with Polycystic Ovary Syndrome (PCOS) is also well acknowledged (Diamanti-Kandarakis et al.; Therapeutic effects of metformin on insulin resistance and hyperandrogenism in polycystic ovary syndrome; *European Journal of Endocrinology* (1998) 138 269-274, Andrea Dunaif; Insulin Resistance and the Polycystic Ovary Syndrome : Mechanism and Implications for Pathogenesis; *Endocrine Reviews* 18(6): 774-800).

The independent risk factors obesity and hypertension for atherosclerotic diseases are also associated with insulin resistance. Using a combination of insulin/glucose clamps, tracer glucose infusion and indirect calorimetry, it was demonstrated that the insulin resistance of essential hypertension is located in peripheral tissues (principally muscle) and correlates directly with the severity of hypertension (DeFronzo and Ferrannini, *Diabetes Care* 1991, 14, 173). In hypertension of obese people, insulin resistance generates hyperinsulinemia, which is recruited as a mechanism to limit further weight gain via thermogenesis, but insulin also increases renal sodium re-absorption and stimulates the sympathetic nervous system in kidneys, heart, and vasculature, creating hypertension.

It is assumed that insulin resistance is usually the result of a defect in the insulin receptor signaling system, at a site post binding of insulin to the receptor. Accumulated scientific evidence demonstrating insulin resistance in the major tissues which respond to insulin (muscle, liver, adipose), strongly suggests that a defect in insulin signal transduction resides at an early step in this cascade, specifically at the insulin receptor kinase activity, which appears to be diminished (Mounib Elchebly, Alan Cheng, Michel L. Tremblay; Modulation of insulin signaling by protein tyrosine phosphatases; *J.Mol.Med.* (2000) 78:473-482).

Protein-tyrosine phosphatases (PTPs) play an important role in the regulation of phosphorylation of proteins and represent the counterparts of kinases. Among classical PTPs, there are two types : (i) non-receptor or intracellular PTPs; and (ii) receptor-like PTPs. Most intracellular PTPs contain one catalytic domain only, whereas most receptor-like enzymes contain two. The catalytic domain consists of about 250 amino acids (Niels Peter Hundahl Moller et al. Protein tyrosine phosphatases (PTPs) as drug targets: Inhibitors of PTP-1B for the treatment of diabetes; *Current Opinion in Drug Discovery & Development* 2000 3(5):527-540).

McGuire et al. (*Diabetes* 1991, 40, 939), demonstrated that non-diabetic glucose intolerant subjects possessed significantly elevated levels of PTP activity in muscle tissue vs. normal subjects, and that insulin infusion failed to suppress PTP activity as it did in insulin sensitive subjects.

The interaction of insulin with its receptor leads to phosphorylation of certain tyrosine molecules within the receptor protein, thus activating the receptor kinase. PTPs dephosphorylate the activated insulin receptor, attenuating the tyrosine kinase activity. PTPs can also modulate post-receptor signaling by catalyzing the dephosphorylation of cellular substrates of the insulin receptor kinase. The enzymes that appear most likely to closely associate with the insulin receptor and therefore, most likely to regulate the insulin receptor kinase activity, include PTP1B, LAR, PTP-alpha. and SH-PTP2 (Lori Klaman et al.; Increased Energy Expenditure, Decreased Adiposity, and Tissue-specific Insulin Sensitivity in Protein-Tyrosine Phosphatase 1B-Deficient Mice; *Molecular and Cellular Biology* 2000, 5479-5489).

The insulin receptor (IR) is a prototypical tyrosine kinase receptor whose ligand binding and dimerization results in auto-phosphorylation on multiple tyrosines. This is followed by the recruitment and phosphorylation of IRS1-4 (depending on the tissue) and PI3K. Although vanadium-containing compounds have been known since the 19^{th} century to alleviate diabetes, it was understood only recently that these inhibitors stimulate the insulin signaling pathway by blocking PTP action. Evidence for the involvement of the IR (insulin receptor) and IRS-1 in this phenotype was that both proteins show increased tyrosine phosphorylation in the PTP1B-mutated mice. The available data strongly suggest that in particular PTP1B is a promising target for the development of drugs to treat diabetes and obesity (Brian P. Kennedy and Chidambaram Ramachandran; Protein Tyrosine Phosphatase-1B in Diabetes; *Biochemical Pharmacology,* Vol. 60, pp. 877-883, 2000).

Meyerovitch et al (*J. Clinical Invest.* 1989, 84, 976) observed significantly increased PTP activity in the livers of two rodent models of IDDM, the genetically diabetic BB rat, and the STZ-induced diabetic rat. Sredy et al (*Metabolism,* 44, 1074, 1995) observed similar increased PTP activity in the livers of obese, diabetic ob/ob mice, a genetic rodent model of NIDDM.

Krogdal et al. (WO 00/54771) discloses 3,5-pyrazolidinedione derivatives to combat viral infections.

Kato Fuminori et al (JP-10045588) reports alpha glucosidase inhibitors containing a pyrazolone compound. Such compounds are said to be useful among others for the treatment of diabetes mellitus.

L.A. Voronkova (Russian Pharmacol. And Toxic. (1973(), Vol. 36, No. 6, p. 235, reports the hypoglycemic action of 1,2-di(p-sulfamidophenyl-4-butyl pyrazolidone-3,5).

In US-4,591,594 as well as in EP-028,410 1,2-diphenyl-4-[2-[phenylsulfinyl]ethyl]-3,5-pyrazolidinedione is said to have an anti-obesity effect.

The compounds of this invention have been shown to inhibit PTPs. They are useful in the treatment of insulin resistance associated with obesity, glucose intolerance, diabetes mellitus, hypertension and ischemic diseases of the large and small blood vessels.

### Summary of the invention

The present invention is related to the use of alkylidene pyrazolidinedione derivatives of formula (I) for the treatment and/or prevention of diabetes type I and/or II, obesity and polycystic ovary syndrome (PCOS).

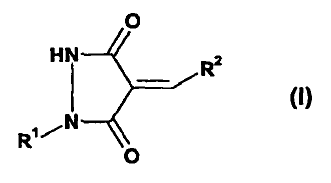

The present invention is furthermore related to novel alkylidene pyrazolidinedione derivatives.

### Detailed description of the invention

The following paragraphs provide definitions of the various chemical moieties that make up the compounds according to the invention and are intended to apply uniformly throughout the specification and claims unless an otherwise expressly set out definition provides a broader definition.

"PTPs" are protein tyrosine phosphatases and include for instance PTP1B, TC-PTP, PTP-β, DEP-1, LAR, SHP-1, SHP-2, GLEPP-1, PTP-κ, PTP-µ, VHR, hVH5, LMW-PTP, PTEN.

"C₁-C₆ -alkyl" refers to monovalent alkyl groups having 1 to 6 carbon atoms. This term is exemplified by groups such as methyl, ethyl, n-propyl, isopropyl, n-butyl, isobutyl, *tert-*butyl, n-hexyl.

"Aryl" refers to an unsaturated aromatic carbocyclic group of from 6 to 14 carbon atoms having a single ring (*e.g.,* phenyl) or multiple condensed rings (*e.g.*, naphthyl). Preferred aryl include phenyl, naphthyl, phenantrenyl.

"C₁-C₆-alkyl aryl" refers to C₁-C₆-alkyl groups having an aryl substituent, including benzyl, phenethyl.

"Heteroaryl" refers to a monocyclic heteroaromatic, or a bicyclic or a tricyclic fused-ring heteroaromatic group. Particular examples of heteroaromatic groups include optionally substituted pyridyl, pyrrolyl, furyl, thienyl, imidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl,1,3,4-triazinyl, 1,2,3-triazinyl, benzofuryl, [2,3-dihydro]benzofuryl, isobenzofuryl, benzothienyl, benzotriazolyl, isobenzothienyl, indolyl, isoindolyl, 3H-indolyl, benzimidazolyl, imidazo[1,2-a]pyridyl, benzothiazolyl, benzoxazolyl, quinolizinyl, quinazolinyl, pthalazinyl, quinoxalinyl, cinnolinyl, napthyridinyl, pyrido[3,4-b]pyridyl, pyrido[3,2-b]pyridyl, pyrido[4,3-b]pyridyl, quinolyl, isoquinolyl, tetrazolyl, 5,6,7,8-tetrahydroquinolyl, 5,6,7,8-tetrahydroisoquinolyl, purinyl, pteridinyl, carbazolyl, xanthenyl or benzoquinolyl.

"C₁-C₆-alkyl heteroaryl" refers to C₁-C₆-alkyl groups having a heteroaryl substituent, including 2-furylmethyl, 2-thienylmethyl, 2-(1H-indol-3-yl)ethyl.

"C₂-C₆-alkenyl" refers to alkenyl groups having from 2 to 6 carbon atoms and having at least 1 or 2 sites of alkenyl unsaturation. Preferable alkenyl groups include ethenyl (-CH=CH₂), n-2-propenyl (allyl, -CH₂CH=CH₂).

"C₂-C₆-alkenyl aryl" refers to C₂-C₆-alkenyl groups having an aryl substituent, including 2-phenylvinyl.

"C₂-C₆-alkenyl heteroaryl" refers to C₂-C₆-alkenyl groups having a heteroaryl substituent, including 2-(3-pyridinyl)vinyl.

"C₂-C₆-alkynyl" refers to alkynyl groups having from 2 to 6 carbon atoms and having at least 1-2 sites of alkynyl unsaturation, preferred alkynyl groups include ethynyl (-C≡CH), propargyl (-CH₂C≡CH).

"C₂-C₆-alkynyl aryl" refers to C₂-C₆-alkynyl groups having an aryl substituent, including phenylethynyl.

"C₂-C₆-alkynyl heteroaryl" refers to C₂-C₆-alkynyl groups having a heteroaryl substituent, including 2-thienylethynyl.

"C₃-C₈-cycloalkyl" refers to a saturated carbocyclic group of from 3 to 8 carbon atoms having a single ring (*e.g.*, cyclohexyl) or multiple condensed rings (*e.g.*, norbornyl). Preferred cycloalkyl include cyclopentyl, cyclohexyl, norbornyl.

"C₁-C₆-alkyl cycloalkyl" refers to C₁-C₆-alkyl groups having a cycloalkyl substituent, including cyclohexylmethyl, cyclopentylpropyl.

"heterocycloalkyl" refers to a C₃-C₈-cycloalkyl group according to the definition above, in which up to 3 carbon atoms are replaced by heteroatoms chosen from the group consisting of O, S, NR, R being defined as hydrogen or methyl. Preferred heterocycloalkyl include pyrrolidine, piperidine, piperazine, 1-methylpiperazine, morpholine.

"C₁-C₆-alkyl heterocycloalkyl" refers to C₁-C₆-alkyl groups having a heterocycloalkyl substituent, including 2-(1-pyrrolidinyl)ethyl, 4-morpholinylmethyl, (1-methyl-4-piperidinyl)methyl.

"Carboxy" refers to the group -C(O)OH.

"C₁-C₆-alkyl carboxy" refers to C₁-C₆-alkyl groups having a carboxy substituent, including 2-carboxyethyl.

"Acyl" refers to the group -C(O)R where R includes H, "C₁-C₆-alkyl", "C₂-C₆-alkenyl", "C₂-C₆-alkynyl", "C₃-C₈-cycloalkyl", heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂₋C₆-alkynyl aryl", "C₂-C₆-alkynylheteroaryl", "C₁-C₆-alkyl cycloalkyl", "C₁-C₆-alkyl heterocycloalkyl".

"C₁-C₆-alkyl acyl" refers to C₁-C₆-alkyl groups having an acyl substituent, including 2-acetylethyl.

"Aryl acyl" refers to aryl groups having an acyl substituent, including 2-acetylphenyl.

"Heteroaryl acyl" refers to hetereoaryl groups having an acyl substituent, including 2-acetylpyridyl.

"C₃-C₈-(hetero)cycloalkyl acyl" refers to 3 to 8 membered cycloalkyl or heterocycloalkyl groups having an acyl substituent.

"Acyloxy" refers to the group -OC(O)R where R includes H, "C₁-C₆-alkyl", "C₂-C₆₋alkenyl", "C₂-C₆-alkynyl", "C₃-C₈-cycloalkyl", heterocycloalkyl"heterocycloalkyl", "aryl", "heteroaryl", "C₁-C₆-alkyl aryl" or "C₁-C₆-alkyl heteroaryl", "C₂-C₆-alkenyl aryl", "C₂-C₆₋alkenyl heteroaryl", "C₂-C₆-alkynyl aryl", "C₂-C₆-alkynylheteroaryl", "C₁-C₆-alkyl cycloalkyl", "C₁-C₆-alkyl heterocycloalkyl".

"C₁-C₆-alkyl acyloxy" refers to C₁-C₆-alkyl groups having an acyloxy substituent, including 2-(acetyloxy)ethyl.

"Alkoxy" refers to the group -O-R where R includes "C₁-C₆-alkyl", "C₂-C₆-alkenyl", "C₂₋C₆-alkynyl", "C₃-C₈-cycloalkyl", "heterocycloalkyl" "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂₋C₆-alkynyl aryl", "C₂-C₆-alkynylheteroaryl", "C₁-C₆-alkyl cycloalkyl", "C₁-C₆-alkyl heterocycloalkyl".

"C₁-C₆-alkyl alkoxy" refers to C₁-C₆-alkyl groups having an alkoxy substituent, including 2-ethoxyethyl.

"Alkoxycarbonyl" refers to the group -C(O)OR where R includes "C₁-C₆-alkyl", "C₂-C₆₋alkenyl", "C₂-C₆-alkynyl", "C₃-C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆-alkynylheteroaryl", "C₁-C₆-alkyl cycloalkyl", "C₁-C₆-alkyl heterocycloalkyl".

"C₁-C₆-alkyl alkoxycarbonyl" refers to C₁-C₆-alkyl groups having an alkoxycarbonyl substituent, including 2-(benzyloxycarbonyl)ethyl.

"Aminocarbonyl" refers to the group -C(O)NRR' where each R, R' includes independently hydrogen, "C₁-C₆-alkyl", "C₂-C₆-alkenyl", "C₂-C₆-alkynyl", "C₃-C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆₋alkynylheteroaryl", "C₁-C₆-alkyl cycloalkyl", "C₁-C₆-alkyl heterocycloalkyl".

"C₁-C₆-alkyl aminocarbonyl" refers to C₁-C₆-alkyl groups having an aminocarbonyl substituent, including 2-(dimethylaminocarbonyl)ethyl.

"Acylamino" refers to the group NRC(O)R' where each R, R' is independently hydrogen, "C₁-C₆-alkyl", "C₂-C₆-alkenyl", "C₂-C₆-alkynyl", "C₃-C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆-alkynylheteroaryl", "C₁-C₆-alkyl cycloalkyl", "C₁-C₆-alkyl heterocycloalkyl".

"C₁-C₆-alkyl acylamino" refers to C₁-C₆-alkyl groups having an acylamino substituent, including 2-(propionylamino)ethyl.

"Ureido" refers to the group -NRC(O)NR'R" where each R, R', R" is independently hydrogen, "C₁-C₆-alkyl", "C₂-C₆-alkenyl", "C₂-C₆-alkynyl", "C₃-C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆₋alkynylheteroaryl", "C₁-C₆-alkyl cycloalkyl", "C₁-C₆-alkyl heterocycloalkyl", and where R' and R", together with the nitrogen atom to which they are attached, can optionally form a 3-8-membered heterocycloalkyl ring.

"C₁-C₆-alkyl ureido" refers to C₁-C₆-alkyl groups having an ureido substituent, including 2-(*N*'-methylureido)ethyl.

"Carbamate" refers to the group NRC(O)OR' where each R, R' is independently hydrogen, "C₁-C₆-alkyl", "C₂-C₆-alkenyl", "C₂-C₆-alkynyl", "C₃-C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆₋alkynylheteroaryl", "C₁-C₆-alkyl cycloalkyl", "C₁-C₆-alkyl heterocycloalkyl".

"Amino" refers to the group -NRR' where each R, R' is independently hydrogen, "C₁-C₆₋alkyl", "C₂-C₆-alkenyl", "C₂-C₆-alkynyl", "C₃-C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆₋alkenyl heteroaryl", "C₂-C₆-alkynyl aryl", "C₂-C₆-alkynylheteroaryl", "C₁-C₆-alkyl cycloalkyl", "C₁-C₆-alkyl heterocycloalkyl", and where R and R', together with the nitrogen atom to which they are attached, can optionally form a 3-8-membered heterocycloalkyl ring.

"C₁-C₆-alkyl amino" refers to C₁-C₆-alkyl groups having an amino substituent, including 2-(1-pyrrolidinyl)ethyl.

"Ammonium" refers to a positively charged group -N⁺RR'R", where each R, R',R" is independently, "C₁-C₆-alkyl", "C₂-C₆-alkenyl", "C₂-C₆-alkynyl", "C₃-C₈-cycloalkyl", "heterocycloalkyl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆-alkynylheteroaryl", "C₁-C₆-alkyl cycloalkyl", "C₁-C₆-alkyl heterocycloalkyl", and where R and R', together with the nitrogen atom to which they are attached, can optionally form a 3-8-membered heterocycloalkyl ring.

"C₁-C₆-alkyl ammonium" refers to C₁-C₆-alkyl groups having an ammonium substituent, including 2-(1-pyrrolidinyl)ethyl.

"Halogen" refers to fluoro, chloro, bromo and iodo atoms.

"Sulfonyloxy" refers to a group -OSO₂-R wherein R is selected from H, "C₁-C₆-alkyl", "C₁-C₆-alkyl" substituted with halogens, *e.g.*, an -OSO₂-CF₃ group, "C₂-C₆-alkenyl", "C₂₋C₆-alkynyl", "C₃-C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂₋C₆-alkynyl aryl", "C₂-C₆-alkynylheteroaryl", "C₁-C₆-alkyl cycloalkyl", "C₁-C₆-alkyl heterocycloalkyl".

"C₁-C₆-alkyl sulfonyloxy" refers to C₁-C₆-alkyl groups having a sulfonyloxy substituent, including 2-(methylsulfonyloxy)ethyl.

"Sulfonyl" refers to group "-SO₂-R" wherein R is selected from H, "aryl", "heteroaryl", "C₁-C₆-alkyl", "C₁-C₆-alkyl" substituted with halogens, *e.g.*, an -SO₂-CF₃ group, "C₂-C₆₋alkenyl", "C₂-C₆-alkynyl", "C₃-C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆-alkynylheteroaryl", "C₁-C₆-alkyl cycloalkyl", "C₁-C₆-alkyl heterocycloalkyl".

"C₁-C₆-alkyl sulfonyl" refers to C₁-C₆-alkyl groups having a sulfonyl substituent, including 2-(methylsulfonyl)ethyl.

"Sulfinyl" refers to a group "-S(O)-R" wherein R is selected from H, "C₁-C₆-alkyl", "C₁₋C₆-alkyl" substituted with halogens, e.g., an -SO-CF₃ group, "C₂-C₆-alkenyl", "C₂-C₆₋alkynyl", "C₃-C₈-cycloalkyl", "heterocycloalkyl", "aryl", "heteroaryl" "C₁-C₆-alkyl aryl" or "C₁-C₆-alkyl heteroaryl", "C₂-C₆-alkenyl aryl", "C₂-C₆-alkenyl heteroaryl", "C₂-C₆₋alkynyl aryl", "C₂-C₆-alkynylheteroaryl", "C₁-C₆-alkyl cycloalkyl", "C₁-C₆-alkyl heterocycloalkyl".

"C₁-C₆-alkyl sulfinyl" refers to C₁-C₆-alkyl groups having a sulfinyl substituent, including 2-(methylsulfinyl)ethyl.

"Sulfanyl" refers to groups -S-R where R includes H, "C₁-C₆-alkyl", "C₁-C₆-alkyl" substituted with halogens, e.g., an -SO-CF₃ group, "C₂-C₆-alkenyl", "C₂-C₆-alkynyl", "C₃₋C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂₋C₆-alkynylheteroaryl", "C₁-C₆-alkyl cycloalkyl", "C₁-C₆-alkyl heterocycloalkyl". Preferred sulfanyl groups include methylsulfanyl, ethylsulfanyl.

"C₁-C₆-alkyl sulfanyl" refers to C₁-C₆-alkyl groups having a sulfanyl substituent, including 2-(ethylsulfanyl)ethyl.

"Sulfonylamino" refers to a group NRSO₂-R' where each R, R' includes independently hydrogen, "C₁-C₆-alkyl", "C₂-C₆-alkenyl", "C₂-C₆-alkynyl ", "C₃-C₈-cycloalkyl", "heterocycloalkyl" "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆₋alkynylheteroaryl", "C₁-C₆-alkyl cycloalkyl", "C₁-C₆-alkyl heterocycloalkyl".

"C₁-C₆-alkyl sulfonylamino" refers to C₁-C₆-alkyl groups having a sulfonylamino substituent, including 2-(ethylsulfonylamino)ethyl.

"Aminosulfonyl" refers to a group -SO₂-NRR' where each R, R' includes independently hydrogen, "C₁-C₆-alkyl", "C₂-C₆-alkenyl", "C₂-C₆-alkynyl", "C₃-C₈-cycloalkyl", "heterocycloalkyl", "aryl", "heteroaryl", "C₁-C₆-alkyl aryl" or "C₁-C₆-alkyl heteroaryl", "C₂-C₆-alkenyl aryl", "C₂-C₆-alkenyl heteroaryl", "C₂-C₆-alkynyl aryl", "C₂-C₆₋alkynylheteroaryl", "C₁-C₆-alkyl cycloalkyl", "C₁-C₆-alkyl heterocycloalkyl".

"C₁-C₆-alkyl aminosulfonyl" refers to C₁-C₆-alkyl groups having an aminosulfonyl substituent, including 2-(cyclohexylaminosulfonyl)ethyl.

"Substituted or unsubstituted" : Unless otherwise constrained by the definition of the individual substituent, the above set out groups, like "alkyl", like trihalomethyl, "alkenyl", "alkynyl", "aryl" and "heteroaryl" etc. groups can optionally be substituted with from 1 to 5 substituents selected from the group consisting of "C₁-C₆-alkyl", "C₂-C₆-alkenyl", "C₂₋C₆-alkynyl", "cyclo-alkyl", "heterocycloalkyl", "C₁-C₆-alkyl aryl", "C₁-C₆-alkyl heteroaryl", "C₁-C₆-alkyl cycloalkyl", "C₁-C₆-alkyl heterocycloalkyl", "amino", "ammonium", "acyl", "acyloxy", "acylamino", "aminocarbonyl", "alkoxycarbonyl", "ureido", "carbamate", "aryl", "heteroaryl", "sulfinyl", "sulfonyl", "alkoxy", "sulfanyl", "halogen", "carboxy", cyano, hydroxy, mercapto, nitro. Alternatively said substitution could also comprise situations where neighbouring substituents have undergone ring closure, notably when vicinal functional substituents are involved, thus forming, *e.g.*, lactams, lactons, cyclic anhydrides, but also acetals, thioacetals, aminals formed by ring closure for instance in an effort to obtain a protective group.

"Pharmaceutically acceptable salts or complexes" refers to salts or complexes of the below-identified compounds of formula (I) that retain the desired biological activity. Examples of such salts include acid addition salts formed with inorganic acids (e.g. hydrochloric acid, hydrobromic acid, sulfuric acid, phosphoric acid, nitric acid, and the like), and salts formed with organic acids such as acetic acid, oxalic acid, tartaric acid, succinic acid, malic acid, fumaric acid, maleic acid, ascorbic acid, benzoic acid, tannic acid, pamoic acid, alginic acid, polyglutamic acid, naphthalene sulfonic acid, naphthalene disulfonic acid, and poly-galacturonic acid. Said compounds can also be administered as pharmaceutically acceptable quaternary salts known by a person skilled in the art, which specifically include the quarternary ammonium salt of the formula -NR,R',R" ⁺ Z⁻, wherein R, R', R" is independently hydrogen, alkyl, or benzyl, and Z is a counterion, including chloride, bromide, iodide, -O-alkyl, toluenesulfonate, methylsulfonate, sulfonate, phosphate, or carboxylate (such as benzoate, succinate, acetate, glycolate, maleate, malate, fumarate, citrate, tartrate, ascorbate, cinnamoate, mandeloate, and diphenylacetate).

"Pharmaceutically active derivative" refers to any compound that upon administration to the recipient, is capable of providing directly or indirectly, the activity disclosed herein.

"Enantiomeric excess" (ee) refers to the products that are obtained by an asymmetric synthesis, i.e. a synthesis involving non-racemic starting materials and/or reagents or a synthesis comprising at least one enantioselective step, whereby a surplus of one enantiomer in the order of at least about 52% ee is yielded. In the absence of an asymmetric synthesis, racemic products are usually obtained that do however also have the inventive set out activity as PTP inhibitors.

The compounds of the present invention are those of formula (I) and/or its tautomers of formula (II).

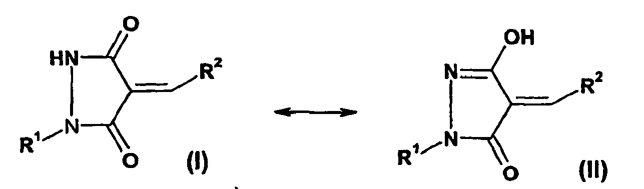

In said formulae (I) or (II), R¹ is an unsubstituted or substituted aryl and R² represent an unsubstituted or substituted aryl or heteroaryl.

Further to the tautomers of formula (II), the present invention also comprises the geometrical isomers of formula (I), its optically active forms as enantiomers, diastereomers and its racemate forms, as well as pharmaceutically acceptable salts thereof. Preferred pharmaceutically acceptable salts of the formula (I) are acid addition salts formed with pharmaceutically acceptable acids like hydrochloride, hydrobromide, sulfate or bisulfate, phosphate or hydrogen phosphate, acetate, benzoate, succinate, fumarate, maleate, lactate, citrate, tartrate, gluconate, methanesulfonate, benzenesulfonate, and *para*-toluenesulfonate salts.

Thus, upon taking into account the tautomers and their geometrical isomers the compounds used for the present invention have the following formulae :

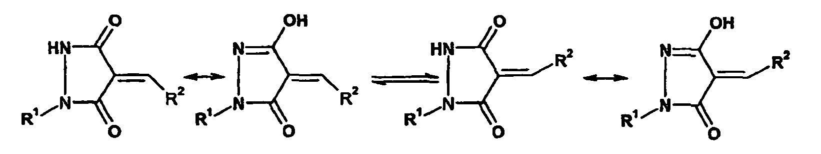

R² may be selected from the group consisting of optionally substituted phenyl or naphthyl, phenantrenyl, pyridyl, pyrrolyl, furyl, thienyl, imidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, 1,3,4-triazinyl, 1,2,3-triazinyl, benzofuryl, [2,3-dihydro]benzofuryl, isobenzofuryl, benzothienyl, benzotriazolyl, isobenzothienyl, indolyl, isoindolyl, 3H-indolyl, benzimidazolyl, imidazo-[1,2-a]pyridyl, benzothiazolyl, benzoxazolyl, quinolizinyl, quinazolinyl, pthalazinyl, quinoxalinyl, cinnolinyl, napthyridinyl, pyrido[3,4-b]pyridyl, pyrido[3,2-b]pyridyl, pyrido[4,3-b]pyridyl, quinolyl, isoquinolyl, tetrazolyl, 5,6,7,8-tetrahydroquinolyl, 5,6,7,8-tetrahydroisoquinolyl, purinyl, pteridinyl, carbazolyl, xanthenyl or benzoquinolyl.

According to a preferred embodiment, R² is an unsubstituted or substituted furanyl, thienyl, pyrrolyl, indolyl, quinolyl or carbazolyl. Such aryl or heteroaryl moieties may be substituted by one or two substituents preferably selected from the group comprising or consisting of substituted or unsubstituted C₁-C₆ alkyl (e.g. methyl or ethyl), substituted or unsubstituted C₂-C₆ alkenyl, substituted or unsubstituted C₂-C₆ alkynyl, substituted or unsubstituted aryl or heteroaryl, substituted or unsubstituted 3-8 membered cycloalkyl or heterocycloalkyl, amino, sulfonyl, sulfanyl, sulfinyl, C₁-C₆-alkyl sulfonyl, C₁-C₆-alkyl sulfinyl, C₁-C₆-alkyl sulfanyl, alkoxy, halogen, e.g. an iodo, cyano, carboxy, alkoxycarbonyl, or nitro group.

More preferred is where R² is a furanyl, thienyl, pyrrolyl, indolyl, quinolyl or carbazolyl moiety which is optionally substituted by a halogen, carboxy, alkoxycarbonyl or nitro group.

A more preferred moiety R² is an unsubstituted or substituted furanyl.

Alternatively, R² may be a substituted phenyl or napthyl which is substituted by halogen, e.g. an iodo, cyano, carboxyl, amino or nitro group.

According to a preferred embodiment, R¹ is a substituted phenyl, e.g. a halogeno phenyl group, in particular, R¹ is a iodophenyl, trifluoromethylphenyl, chloro-methylphenyl, iodobenzoic acid methyl ester, or a biphenyl group.

A further aspect of the present invention is related to the use of the alkylidene pyrazolidine-dione derivatives according to formula (I) for the preparation of pharmaceutical compositions for the treatment and/or prevention of diabetes type I and/or II, obesity, polycystic ovary syndrome (PCOS).

Specific compounds which are may be used for such treatment are the following (compounds are a E/Z mixture, where not otherwise specified):
4-(4-Iodo-benzylidene)-1-(4-iodo-phenyl)-pyrazolidine-3,5-dione
4-(Dimethyl-furan-2-ylmethylene)-1-(3-trifluoromethyl-phenyl)-pyrazolidine-3,5-dione
1-Biphenyl-4-yl-4-furan-2-ylmethylene-pyrazolidine-3,5-dione
4-(2-Hydroxy-benzylidene)-1-(4-iodo-phenyl)-pyrazolidine-3,5-dione
4-Furan-2-ylmethylene-1-(4-iodo-phenyl)-pyrazolidine-3,5-dione
4-(5-Bromo-2-hydroxy-benzylidene)-1-(4-iodo-phenyl)-pyrazolidine-3,5-dione
4-(3-Iodo-benzylidene)-1-(4-iodo-phenyl)-pyrazolidine-3,5-dione
4-Benzylidene-1-(4-iodo-phenyl)-pyrazolidine-3,5-dione
4-(4-Dimethylamino-benzylidene)-1-(4-iodo-phenyl)-pyrazolidine-3,5-dione
4-[1-(4-Iodo-phenyl)-3,5-dioxo-pyrazolidin-4-ylidenemethyl]-benzoic acid methyl ester
1-(4-Iodo-phenyl)-4-thiophen-2-ylmethylene-pyrazolidine-3,5-dione
4-[1-(4-Iodo-phenyl)-3,5-dioxo-pyrazolidin-4-ylidenemethyl]-benzoic acid
{4-[1-(4-Iodo-phenyl)-3,5-dioxo-pyrazolidin-4-ylidenemethyl]-phenoxy}-acetic acid
(E)-3-{4-[1-(4-Iodo-phenyl)-3,5-dioxo-pyrazolidin-4-ylidenemethyl]-phenyl}-acrylic acid
2-Hydroxy-5-[1-(4-iodo-phenyl)-3,5-dioxo-pyrazolidin-4-ylidenemethyl]-benzoic acid
3-[1-(4-Iodo-phenyl)-3,5-dioxo-pyrazolidin-4-ylidenemethyl]-benzoic acid
(E)-3-{4-[3,5-Dioxo-1-(3-trifluoromethyl-phenyl)-pyrazolidin-4-ylidenemethyl]-phenyl}-acrylic acid
{2-[1-(4-Iodo-phenyl)-3,5-dioxo-pyrazolidin-4-ylidenemethyl]-phenoxy}-acetic acid
{4-[3,5-Dioxo-1-(3-trifluoromethyl-phenyl)-pyrazolidin-4-ylidenemethyl]-phenoxy}-acetic acid
5-[3,5-Dioxo-1-(3-trifluoromethyl-phenyl)-pyrazolidin-4-ylidenemethyl]-2-hydroxybenzoic acid
{2-[3,5-Dioxo-1-(3-trifluoromethyl-phenyl)-pyrazolidin-4-ylidenemethyl]-phenoxy} -acetic acid
Acetic acid 5-[1-(4-iodo-phenyl)-3,5-dioxo-pyrazolidin-4-ylidenemethyl]-furan-2-ylmethyl ester
4-(4-Bromo-furan-2-ylmethylene)-1-(4-iodo-phenyl)-pyrazolidine-3,5-dione
1-(4-Iodo-phenyl)-4-(5-methyl-furan-2-ylmethyl ene)-pyrazolidine-3, 5-dione
4-(5-Bromo-furan-2-ylmethylene)-1-(4-iodo-phenyl)-pyrazolidine-3,5-dione
1-(4-Iodo-phenyl)-4-[5-(2-methoxy-phenyl)-thiophen-2-ylmethylene]-pyrazolidine-3,5-dione
1-(4-Iodo-phenyl)-4-thiophen-3-ylmethylene-pyrazolidine-3,5-dione
1-(4-Iodo-phenyl)-4-(5-phenyl-thiophen-2-ylmethylene)-pyrazolidine-3,5-dione
4-Furan-2-ylmethylene-1-(3-trifluoromethyl-phenyl)-pyrazolidine-3,5-dione
4-(5-Methyl-furan-2-ylmethylene)-1-(3-trifluoromethyl-phenyl)-pyrazolidine-3,5-dione
4-Thiophen-2-ylmethylene-1-(3-trifluoromethyl-phenyl)-pyrazolidine-3,5-dione
4-(4-Bromo-furan-2-ylmethylene)-1-(3-trifluoromethyl-phenyl)-pyrazolidine-3,5-dione
Acetic acid 5-[3,5-dioxo-1-(3-trifluoromethyl-phenyl)-pyrazolidin-4-ylidenemethyl]-furan-2-ylmethyl ester
1-(3-Chloro-4-methyl-phenyl)-4-furan-2-ylmethylene-pyrazolidine-3,5-dione
4-(7-Bromo-8-hydroxy-quinolin-5-ylmethylene)-1-(4-iodo-phenyl)-pyrazolidine-3, 5-dione
1-(4-Iodo-phenyl)-4-[5-(4-nitro-phenyl)-furan-2-ylmethylene]-pyrazolidine-3,5-dione
1 -(4-Iodo-phenyl)-4-[5-(2-nitro-phenyl)-faran-2-ylmethylene]-pyrazolidine-3,5-dione
4-(5-Hydroxymethyl-furan-2-ylmethylene)-1-(4-iodo-phenyl)-pyrazolidine-3,5-dione
4-((E)-3-Furan-2-yl-allylidene)-1-(4-iodo-phenyl)-pyrazolidine-3,5-dione
4-Furan-3-ylmethylene-1-(4-iodo-phenyl)-pyrazolidine-3, 5-dione
1-(4-Iodo-phenyl)-4-(5-nitro-thiophen-2-ylmethylene)-pyrazolidine-3,5-dione
4-(5-Hydroxymethyl-furan-2-ylmethylene)-1-(3-trifluoromethyl-phenyl)-pyrazolidine-3,5-dione
4-Furan-3-ylmethylene-1-(3-trifluoromethyl-phenyl)-pyrazolidine-3,5-dione
4-(5-Nitro-thiophen-2-ylmethylene)-1-(3-trifluoromethyl-phenyl)-pyrazolidine-3,5-dione
1-(4-Iodo-phenyl)-4-(5-nitro-furan-2-ylmethylene)-pyrazolidine-3,5-dione
1-(4-Iodo-phenyl)-4-[5-(3-nitro-phenyl)-furan-2-ylmethylene]-pyrazolidine-3,5-dione
4-(4,5-Dimethyl-furan-2-ylmethylene)-1-(4-iodo-phenyl)-pyrazolidine-3,5-dione
5-[1-(4-Iodo-phenyl)-3,5-dioxo-pyrazolidin-4-ylidenemethyl]-furan-2-sulfonic acid
4-[1-(4-Iodo-phenyl)-3,5-dioxo-pyrazolidin-4-ylidenemethyl]-benzonitrile
1-(4-Iodo-phenyl)-4-(3-methyl-thiophen-2-ylmethylene)-pyrazolidine-3,5-dione
4-(4-Bromo-thiophen-2-ylmethylene)-1-(4-iodo-phenyl)-pyrazolidine-3,5-dione
1-(4-Iodo-phenyl)-4-(5-methyl-thiophen-2-ylmethylene)-pyrazolidine-3,5-dione
4-Benzo[b]thiophen-2-ylmethylene-1-(4-iodo-phenyl)-pyrazolidine-3,5-dione
1-(4-Iodo-phenyl)-4-(4-nitro-thiophen-2-ylmethylene)-pyrazolidine-3, 5-dione
5-[1-(4-Iodo-phenyl)-3,5-dioxo-pyrazolidin-4-ylidenemethyl]-thiophene-3-carboxylic acid
4-[1-(4-Iodo-phenyl)-3,5-dioxo-pyrazolidin-4-ylidenemethyl]-thiophene-3-carboxylic acid
2-[3,5-Dioxo-1-(3-trifluoromethyl-phenyl)-pyrazolidin-4-ylidenemethyl] -thiophene-3-carboxylic acid
4-(4-Nitro-thiophen-2-ylmethylene)-1-(3-trifluoromethyl-phenyl)-pyrazolidine-3,5-dione
4-[3,5-Dioxo-1-(3-trifluoromethyl-phenyl)-pyrazolidin-4-ylidenemethyl]-thiophene-3-carboxylic acid
{3-[3,5-Dioxo-1-(3-trifluoromethyl-phenyl)-pyrazolidin-4-ylidenemethyl]-thiophen-2-ylsulfanyl}-acetic acid
2-[1-(4-Iodo-phenyl)-3,5-dioxo-pyrazolidin-4-ylidenemethyl]-thiophene-3-carboxylic acid
{3-[1-(4-Iodo-phenyl)-3,5-dioxo-pyrazolidin-4-ylidenemethyl]-thiophen-2-ylsulfanyl}-acetic acid
4-(4-Hydroxymethyl-furan-3-ylmethylene)-1-(3-trifluoromethyl-phenyl)-pyrazolidine-3,5-dione
4-benzo[b]thiophen-2-ylmethylene-1-(3-trifluoromethyl-phenyl)-pyrazolidine-3,5-dione
5-[3,5-Dioxo-1-(3-trifluoromethyl-phenyl)-pyrazolidin-4-ylidenemethyl]-thiophene-3-carboxylic acid
5-[1-(4-Iodo-phenyl)-3,5-dioxo-pyrazolidin-4-ylidenemethyl]-furan-2-carboxylic acid
4-[4-(3-Dimethylamino-propylamino)-benzylidene]-1-(4-iodo-phenyl)-pyrazolidine-3,5-dione
1-(4-Iodo-phenyl)-4-(4-pyrrolidin-1-yl-benzylidene)-pyrazolidine-3,5-dione
5-(1-Biphenyl-4-yl-3,5-dioxo-pyrazolidin-4-ylidenemethyl)-furan-2-carboxylic acid
4-(4-Hydroxy-naphthalen-1-ylmethylene)-1-(4-iodo-phenyl)-pyrazolidine-3,5-dione
1-(4-Iodo-phenyl)-4-(1-oxy-pyridin-4-ylmethylene)-pyrazolidine-3,5-dione
2-(4-Furan-2-ylmethylene-3,5-dioxo-pyrazolidin-1-yl)-5-iodo-benzoic acid methyl ester
1-(4-Iodo-phenyl)-4-[2-(4-methyl-piperazin-1-yl)-benzylidene]-pyrazolidine-3,5-dione
4-(1H-Indol-5-ylmethylene)-1-(4-iodo-phenyl)-pyrazolidine-3,5-dione
4-(4-Hydroxymethyl-furan-3-ylmethylene)-1-(4-iodo-phenyl)-pyrazolidine-3,5-dione
1-(4-Iodophenyl)-4-(1H-pyrrol-2-ylmethylene)-pyrazolidine-3,5-dione
1-(4-Iodophenyl)-4-(1-methyl-1H-pyrrol-2-ylmethylene)-pyrazolidine-3,5-dione
1-(4-Iodo-phenyl)-4-[1-(2-nitro-benzyl)-1H-pyrrol-2-ylmethylene]-pyrazolidine-3,5-dione
1-(4-Iodo-phenyl)-4-(4-pyridin-2-yl-benzylidene)-pyrazolidine-3,5-dione
1-(4-Iodo-phenyl-4(1H-pyrazol-3-ylmethylene)-pyrazolidine-3,5-dione
1-(4-Iodo-phenyl-4(1H-pyrazol-3-ylmethylene)-pyrazolidine-3,5-dione
4-(2,3-Dihydro-benzofuran-5-ylmethylene)-1-(4-iodo-phenyl)-pyrazolidine-3,5-dione
1-(4-Iodo-phenyl)-4-quinolin-2-ylmethylene-pyrazolidine-3, 5-dione
1-(4-Iodo-phenyl)-4-quinolin-4-ylmethylene-pyrazolidine-3,5-dione
4-[3-(2-Hydroxy-ethoxy)-benzylidene]-1-(4-iodo-phenyl)-pyrazolidine-3,5-dione
4-[4-(3-Dimethylamino-propoxy)-benzylidene]-1-(4-iodo-phenyl)-pyrazolidine-3,5-dione
1-(4-Iodo-phenyl)-4-(5-naphthalen-2-yl-thiophen-2-ylmethylene)-pyrazolidine-3,5-dione
4-(Diethylamino-hydroxy-benzylidene)-1-(4-iodo-phenyl)-pyrazolidine-3,5-dione
3-{5-[1-(4-Iodo-phenyl)-3,5-dioxo-pyrazolidin-4-ylidenemethyl]-furan-2-yl}-thiophene-2-carboxylic acid methyl ester
1-(4-Iodo-phenyl)-4-[5-(2-nitro-4-trifluoromethyl-phenyl)-furan-2-ylmethylene]-pyrazolidine-3,5-dione
4-(5-Chloro-thiophen-2-ylmethylene)-1-(4-iodo-phenyl)-pyrazolidine-3,5-dione
1-(4-Iodo-phenyl)-4-thiazol-2-ylmethylene-pyrazolidine-3,5-dione
1-(4-Iodo-phenyl)-4-(1-methyl-1H-imidazol-2-ylmethylene)-pyrazolidine-3,5-dione
4-(2-Diethylamino-thiazol-5-ylmethylene)-1-(4-iodo-phenyl)-pyrazolidine-3,5-dione
4-[2-(4-Benzyl-piperazin-1-yl)-thiazol-5-ylmethylene]-1-(4-iodo-phenyl)-pyrazolidine-3,5-dione
1-(4-Iodo-phenyl)-4-[2-(4-methoxy-phenoxy)-thiazol-5-ylmethylene]-pyrazolidine-3,5-dione
4-(9-Ethyl-9H-carbazol-3-ylmethylene)-1-(4-iodo-phenyl)-pyrazolidine-3,5-dione
4-Benzo[b]thiophen-3-ylmethylene-1-(4-iodo-phenyl)-pyrazolidine-3,5-dione

Still a further aspect of the present invention is related to novel alkylidene pyrazolidine-dione derivatives falling under formula I
selected from the group consisting of:
4-(2-Hydroxy-benzylidene)-1-(4-iodo-phenyl)-pyrazolidine-3,5-dione
4-[1-(4-Iodo-phenyl)-3,5-dioxo-pyrazolidin-4-ylidenemethyl]-benzoic acid
{4-[1-(4-Iodo-phenyl)-3,5-dioxo-pyrazolidin-4-ylidenemethyl]-phenoxy}-acetic acid
(E)-3-{4-[1-(4-Iodo-phenyl)-3,5-dioxo-pyrazolidin-4-ylideneimethyl]-phenyl}-acrylic acid
2-Hydroxy-5-[1-(4-iodo-phenyl)-3,5-dioxo-pyrazolidin-4-ylidenemethyl]-benzoic acid
3-[1-(4-Iodo-phenyl)-3,5-dioxo-pyrazolidin-4-ylidenemethyl]-benzoic acid
(E)-3-{4-[3,5-Dioxo-1-(3-trifluoromethyl-phenyl)-pyrazolidin-4-ylidenemethyl]-phenyl}-acrylic acid
{2-[1-(4-Iodo-phenyl)-3,5-dioxo-pyrazolidin-4-ylidenemethyl]-phenoxy}-acetic acid
{4-[3,5-Dioxo-1-(3-trifluoromethyl-phenyl)-pyrazolidin-4-ylidenemethyl]-phenoxy}-acetic acid
5-[3,5-Dioxo-1-(3-trifluoromethyl-phenyl)-pyrazolidin-4-ylidenemethyl]-2-hydroxybenzoic acid
{2-[3,5-Dioxo-1-(3-trifluoromethyl-phenyl)-pyrazolidin-4-ylidenemethyl]-phenoxy}-acetic acid
4-[4-(3-Dimethyl amino-propylamino)-b enzylidene]-1-(4-iodo-phenyl)-pyrazolidine-3, 5-dione
1-(4-Iodo-phenyl)-4-(4-pyrrolidin-1-yl-b enzylidene)-pyrazolidine-3, 5-dione
l-(4-Iodo-phenyl)-4-[2-(4-methyl-piperazin-l-yl)-benzylidene]-pyrazolidine-3,5-dione
1-(4-Iodo-phenyl)-4-(4-pyridin-2-yl-benzylidene)-pyrazolidine-3,5-dione
4-[4-(3-Dimethylamino-propoxy)-benzylidene]-1-(4-iodo-phenyl)-pyrazolidine-3,5-dione
4-(Diethylamino-hydroxy-benzylidene)-1-(4-iodo-phenyl)-pyrazolidine-3,5-dione

The alkylidene pyrazolidinedione derivatives exemplified by the present invention may be prepared from readily available starting materials using the following general methods and procedures. Optimum reaction conditions may vary with the particular reactants or solvents used, but such conditions can be determined by one skilled in the art by routine optimisation procedures.

In general, the alkylidene pyrazolidinedione derivatives according to formula (I) of this invention may be prepared from readily available starting materials. If such starting material is not commercially available, it may be prepared by standard synthetic techniques.

The following general methods and procedures described hereinafter in the Examples may be employed to prepare compounds of formula (I).

Generally, alkylidene pyrazolidinedione derivatives according to formula (III) may be obtained by reacting (Knoevenagel condensation) a pyrazolinedione derivative of formula (IV) with an aldehyde of formula (V), optionally in the presence of a suitable base such as pyridine, NMO, DIEA, TEA in a suitable solvent e.g. ethanol or methanol for several hours, e.g. from 2 to 16 hours. The preferred conditions are heating the pyrazolidinedione of formula (IV) with an aldehyde of formula (V) in ethanol between 60 and 80°C in the presence of catalytic amount of pyridine. The alkylidene pyrazolidinedione of formula (III) could be obtained by deprotection of any of its protected form, e.g. a protected form of the amide (such as a N-benzylamide) according to procedures known by a person skilled in the art. For all the protection, deprotection methods, see Philip J. Kocienski, in *"Protecting Groups",* Georg Thieme Verlag Stuttgart, New York, 1994 and, Theodora W. Greene and Peter G. M. Wuts in *"Protective Groups in Organic Synthesis",* 3^{rd} edition, John Wiley & Sons Inc., 1999 (NY).

The method of preparation of the alkylidene pyrazolidinedione compounds of formula (III) according to the above protocol has the specific advantage of being convenient and economic in the sense that it involves only a few chemical steps.

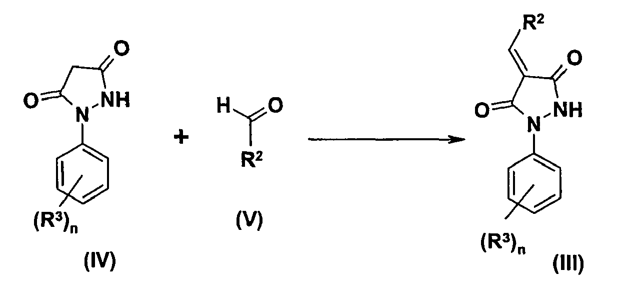

Pyrazolidinedione derivatives according to formula (IV) may be prepared by reacting a phenyl hydrazine derivative according to formula (VI) with a malonic derivative of formula (VII) whereby LG₁ and LG₂ could be any appropriate leaving group. Particularly preferred leaving groups are those selected from the group comprising or consisting of halogen, 1-hydroxy-pyrolidine-2,5-dione, benzotriazol-1-ol, aliphatic or aromatic alkoxy group.

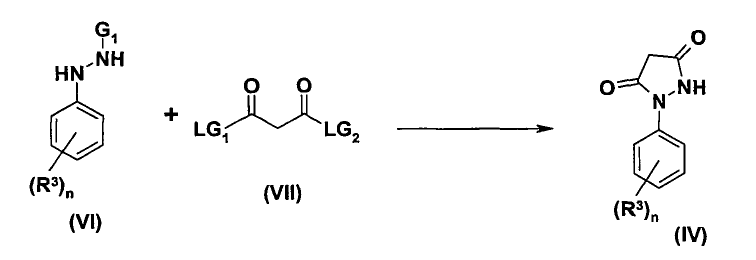

Particularly preferred methods to prepare compounds of formula (IV), i.e. Protocol A, is the condensation of phenyl hydrazine derivatives according to formula (VI) whereby G₁ = H with malonyl dichloride in a suitable solvent such as THF, or with a dialkoxy malonate in a suitable solvent such as ethanol or methanol in presence or absence of a base such as sodium ethoxide or sodium methoxide (Protocol C).

An alternative method of preparation of the pyrazolidinedione derivatives according to formula (IV) consists in the following protocol B, i.e. by reacting a phenyl hydrazine derivative according to formula (VI) whereby G₁ = C(O)CH₃ with malonic derivative of formula (VII) whereby LG₁ and LG₂ are OH in presence of Lewis acid such as PCl₃.

An alternative method of preparation of the pyrazolidinedione derivatives according to formula (IV) may be obtained by the cyclization of compounds of formula (VIII) in the presence of an appropriate base such as sodium ethoxide or methoxide, K₂CO₃, NaH, LDA, LiHDMS, or DIEA in a suitable solvent such as THF or DMF.

Thus, the compounds of formula (VIII) may be obtained by the condensation of a phenyl hydrazine derivatives of formula (VI) whereby G1=H with malonic derivative, whereby LG₁ is any appropriate leaving group such as OH, in presence of coupling reagent (such as DCC, DIC, EDC, PyBOP) or whereby LG₁ is any halogen in presence of a base such as DIEA, TEA or pyridine and LG₂ is an aliphatic or aromatic alkoxy group, or a alkoxy group supported by a polymer. In case that the leaving group LG is an alkoxy group supported by a polymer, compounds of formula (IV), precursor of compounds of formula (III), could then be prepared by a solid phase route as well. The polymer bound malonic derivative could be prepared according to the protocol of Hamper, B. C. *et al. J. Comb. Chem.* **1999**, *1*, 140-150.

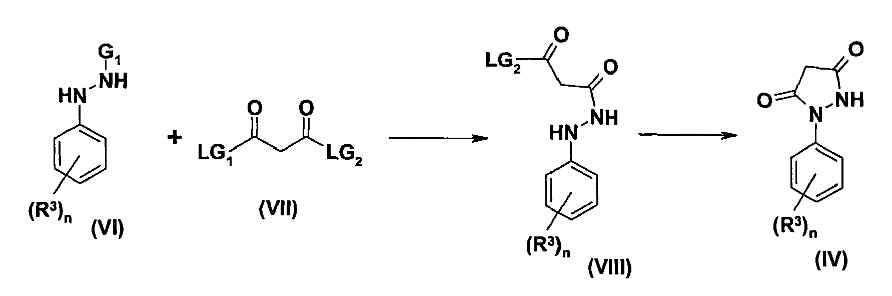

The pyrazolidinedione derivatives according to formula (IV) could furthermore be prepared by alternative methods disclosed by Knabe in J. *et al. Arch. Pharm. (Weinheim)* 1980, *313,* 577-582 (i.e. condensing the phenyl hydrazine derivatives of formula (VI) whereby G1=H with cyanoacetic acid in presence of a coupling reagent such as DCC, EDC, DIC, PyBOP followed by an acid and then a base treatment).

It is also noted that compounds of formula (VI) and (VII) are commercially available compounds or prepared by standard synthetic techniques as hereinafter described in the Examples.

Compounds of formula (I) wherein R¹ is an heteroaryl may be obtained by using corresponding starting compounds (IV) or (VI) having heteroaryl groups according to the methods described above.

When employed as pharmaceuticals, the alkylidene pyrazolidinedione derivatives of the present invention are typically administered in the form of a pharmaceutical composition. Hence, pharmaceutical compositions comprising a compound of formula (I) and a pharmaceutically acceptable carrier, diluent or excipient therefore are also within the scope of the present invention. A person skilled in the art is aware of a whole variety of such carrier, diluent or excipient compounds suitable to formulate a pharmaceutical composition. Also, the present invention provides compounds for use as a medicament.

The compounds of the invention, together with a conventionally employed adjuvant, carrier, diluent or excipient may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous use). Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed.

When employed as pharmaceuticals, the alkylidene pyrazolidinedione derivatives of this invention are typically administered in the form of a pharmaceutical composition. Such compositions can be prepared in a manner well known in the pharmaceutical art and comprise at least one active compound. Generally, the compounds of this invention are administered in a pharmaceutically effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms.

The pharmaceutical compositions of these inventions can be administered by a variety of routes including oral, rectal, transdermal, subcutaneous, intravenous, intramuscular, and intranasal. The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampoules or syringes of the liquid compositions or pills, tablets, capsules in the case of solid compositions. In such compositions, the alkylidene pyrazolidinedione is usually a minor component (from 0.1 to 50% by weight or preferably from 1 to 40% by weight) with the remainder being various vehicles or carriers and processing aids helpful for forming the desired dosing form.

Liquid forms suitable for oral administration may include a suitable aqueous or nonaqueous vehicle with buffers, suspending and dispensing agents, colorants, flavors and the like. Solid forms may include, for example, any of the following ingredients, or compounds of a similar nature: a binder such as microcrystalline cellulose, gum tragacanth or gelatine; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buf fered saline or other injectable carriers known in the art. As above mentioned, the alkylidene pyrazolidinedione derivatives of formula (I) in such compositions is typically a minor component, frequently ranging between 0.05 to 10% by weight with the remainder being the injectable carrier and the like.

The above described components for orally administered or injectable compositions are merely representative. Further materials as well as processing techniques and the like are set out in Part 8 of *Remington's Pharmaceutical Sciences,* 17^{th} Edition, 1985, Marck Publishing Company, Easton, Pennsylvania.

The compounds of this invention can also be administered in sustained release forms or from sustained release drug delivery systems. A description of representative sustained release materials can also be found in *Remington's Pharmaceutical Sciences.*

In the following the present invention shall be illustrated by means of some examples. The following abbreviations are hereinafter used in the accompanying examples: min (minute), h (hour), g (gram), mg (milligram), mmol (millimole), m.p. (melting point), eq (equivalents), mL (milliliter), µL (microliters), mL (milliliters), ACN (Acetonitrile), DBU (Diazabicyclo [5.4.0]undec-7-ene), DIEA (Diisopropylethylamine), CDCl₃ (deuterated chloroform), cHex (Cyclo-hexanes), DCM (Dichloromethane), DIC (Diisopropyl carbodiimide), DMAP (4-Dimethylaminopyridine), Pd(PPh₃)₄ (Tetrakis triphenylphosphine palladium), DMF (Dimethylformamide), DMSO (Dimethylsulfoxide), DMSO-*d*₆ (deuterated dimethylsulfoxide), EDC (1-(3-Dimethyl-amino-propyl)-3-ethylcarbodiimide), EtOAc (Ethyl acetate), Et₂O (Diethyl ether), EtOH (Ethanol), HOBt (1-Hydroxybenzotriazole), K₂CO₃ (potassium carbonate), NaH (Sodium hydride), NaHCO₃ (Sodium bicarbonate), nBuLi (n Butyllithium), TBTU (O-Benzotriazolyl-N,N,N',N'-tetramethyluroniumtetrafluoroborate), TEA (Triethylamine), TFA (Trifluoro-acetic acid), THF (Tetrahydrofuran), MgSO₄ (Magnesium sulfate), PetEther (Petroleum ether), rt (room temperature); **PyBOP®** (Benzotriazole-1-yl-oxy-tris-pyrrolidino-phosphonium hexafluorophosphate); **DCC** (dicyclohexyl carbodiimide); **NMO** (N-Methylmorpholine N-oxide).

### Examples

### Intermediate 1: 1-(4-iodo-phenyl)-pyrazolidine-3,5-dione

### Protocol A

To a cold solution (0°C) of malonyldichloride (705 mg, 5.0 mmol) in 25 mL of dry THF was added a solution of 4-iodophenylhydrazine (1.170 g, 5.0 mmol) in 5 mL of THF) under inert atmosphere. After stirring for 15 min, the mixture was heated at 90°C and stirring was continued overnight. The reaction mixture was cooled to rt and was filtered on a fritté (to remove remaining starting material). The mother liquids were concentrated *in vacuo* and the resulting solid was dissolved in a minimum of hot THF (10 mL). A beige fine powder was precipitated on cooling at 0°C, filtered and dried for 3 days under *vacuo* at 40°C to give 580 mg of 1-(4-iodo-phenyl)-pyrazolidine-3,5-dione (38%).
¹H NMR (DMSO-d₆, 300 MHz) δ 12.5-11 (m, 1H), 7.91 (d, 2H, J=8.3 Hz), 7.48 (d, 2H, J=8.3 Hz), 3.59 (s, 2H).
FI-MS (APCI): m/z (negative mode): 302.0
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 3.80 min (HPLC purity: 98.0%).

### Protocol B

### Step 1 : Preparation of acetic acid N-(4-iodo-phenyl)-hydrazide

To a cold solution of 4-iodophenylhydrazine (1.170 g, 5.0 mmol) in 20 mL of dry DCM acetic anhydride (510 mg, 5.0 mmol) was added under inert atmosphere. After stirring overnight at rt, the solvents were evaporated *in vacuo* to give 1.391 g of acetic acid N-(4-iodo-phenyl)-hydrazide (100 %) as a fine brown powder.
¹H NMR (CDCl₃, 300 MHz) δ 7.51 (d, 2H, J=8.3 Hz), 7.24 (s, 1H), 7.15 (s, 1H), 6.59 (d, 2H, J=8.3 Hz), 2.06 (s, 3H).
FI-MS (APCI): m/z (negative mode): 274.6
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 3.62 min (HPLC purity: 98.5%).

### Step 2 : Preparation of 1-(4-iodo-phenyl)-pyrazolidine-3,5-dione

To acetic acid N-(4-iodo-phenyl)-hydrazide (see below, 276 mg, 1.0 mmol) and malonic acid (156 mg, 1.5 mmol) was added phosphorus trichloride (220 mg, 1.6 mmol) at rt under inert atmosphere. The reaction mixture was stirred at 100°C for 2 h, then cooled to rt. A solution of ice in water was then added (5 mL) and the precipitate was filtered off on a fritté and dried to give 130 mg of 1-(4-iodo-phenyl)-pyrazolidine-3,5-dione (43%) as a light brown fine powder.
The analytical data of the title compound are similar as in protocol A.

### Intermediate 2: 1-(3-trifluoromethyl-phenyl)-pyrazolidine-3,5-dione

Protocol C (Related procedure of Gagnon, P. E. *et al. Can. J. Chem.* **1954,** (*32*), pp823-838) To a solution of 3-(trifluoromethyl)phenylhydrazine (1.76 g, 10.0 mmol) and diethyl malonate (1.60 g, 10.0 mmol) in absolute ethanol (10 mL) was added sodium ethoxide (1.360 g, 20.0 mmol) under inert atmosphere. After stirring the reaction mixture for 24 h at reflux, the excess alcohol was evaporated *in vacuo* and the residue dissolved in water. The aqueous solution was extracted with diethyl ether (3 x) and then acidified with diluted acetic acid (33 %). The precipitate was collected on a fritté and chromatographied on silicagel column using DCM:MeOH (95:5) as eluting solvent. Removal of the solvents in *vacuo* gave 1.28 g of 1-(3-trifluoromethyl-phenyl)-pyrazolidine-3,5-dione as a light yellow fine powder (53 %).
¹H NMR (CDCl₃, 300 MHz) δ 7.82 (s, 1H), 7.72 (d, 1H, J=8.1 Hz), 7.55-7.40 (m, 2H), 3.44 (s, 2H).
FI-MS (APCI): m/z (negative mode): 243.0
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 3.53 min (HPLC purity: 98.0%).

### Intermediate 3 : 1-(3-chloro-4-methyl-phenyl)-pyrazolidine-3,5-dione

To a solution of 3-chloro-p-tolylhydrazine hydrochloride (1.545 g, 8.0 mmol) and diethyl malonate (1.281 g, 8.0 mmol) in absolute ethanol (10 mL) was added sodium ethoxide (1.630 g, 24.0 mmol) under inert atmosphere. After stirring the reaction mixture for 24 h at reflux, the excess alcohol was evaporated *in vacuo* and the residue dissolved in water. The aqueous solutions were extracted with diethyl ether (3 x) and then acidified with diluted acetic acid (33 %). The precipitate was collected on a fritté and chromatographied on silicagel column using DCM:MeOH (94:6) as eluting solvent. Removal of the solvents in *vacuo* gave 1.03 g of 1-(3-chloro-4-methyl-phenyl)-pyrazolidine-3,5-dione as a beige fine powder (58 %).
¹H NMR (DMSO-d₆, 300 MHz) δ 12.6-11 (br s, 1H), 7.72 (s, 1H), 7.51 (d, 1H, J=7.2 Hz), 7.37 (s, 1H, J=8.2 Hz), 3.59 (s, 2H), 2.29 (s, 3H).
FI-MS (APCI): m/z (negative mode): 223.0
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 3.41 min (HPLC purity: 98.0%).

### Intermediate 4: 2-(3,5-dioxo-pyrazolidin-1-yl)-5-iodo-benzoic acid methyl ester

### Step 1 : Preparation of 2-hydrazino-5-iodo-benzoic acid methyl ester hydrochloride

To a cold (-10°C) solution of 2-amino-5-iodo-benzoic acid methyl ester (5.0 g, 18.05 mmol) in an aqueous concentrated HCl solution (20 mL) was added a solution of NaNO₂ (1.245 g, 18.05 mmol) in H₂O (10 mL) at such a rate that the temperature did not exceed 0°C. After stirring at 0°C for 30 min, the reaction mixture was chilled at -10°C and a solution of SnCl₂.2H₂O (14.66 g, 64.98 mmol) in an aqueous concentrated HCl solution (20 mL) was added dropwise at such a rate that the temperature did not exceed -5°C. The resulting cream suspension was warmed to 25°C, filtered and washed with water (2 x 20 °mL), with Et₂O (3 x 30 mL) then with DCM (3 x 30 mL). The product was dried at 45°C for 24h *in vacuo* to give 4.5 g of the 2-hydrazino-5-iodo-benzoic acid methyl ester hydrochloride as a fine brown powder (76%).
¹H NMR (DMSO-d₆, 300 MHz) δ 11.4 (m, 3H), 9.10 (m, 1H), 8.15 (s, 1H), 7.82 (d, 1H, J=8.1 Hz), 7.16 (d, 2H, J=8.1 Hz), 3.91 (s, 3H).
FI-MS (APCI): m/z (negative mode): 258.8 (M-H)-(MeOH)
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 2.84 min (HPLC purity: 98.0%).

### Step 2: Preparation of 2-(3,5-dioxo-pyrazolidin-1-yl)-5-iodo-benzoic acid methyl ester

The same procedure as employed in the preparation of Intermediate 1 (protocol A) but starting from 2-hydrazino-5-iodo-benzoic acid methyl ester hydrochloride (see Step 1). Chromatography on silicagel column using AcOEt as eluting solvent gave a residue. The residue was dissolved in a minimum of hot THF (10 mL). A beige fine powder was precipitated on cooling at 0°C, filtered and dried for 3 days under *vacuo* at 40°C to give 2-(3,5-dioxo-pyrazolidin-1-yl)-5-iodo-benzoic acid methyl ester as a fine white powder (15 %).
¹H NMR (DMSO-d₆, 300 MHz) δ 12.0-11.0 (br s, 1H), 8.00 (s, 1H), 7.98 (dd, 1H, J1=2.2 Hz, J2=7.5 Hz), 7.28 (dd, 1H, J=1.1 Hz, J2=7.5 Hz), 3.75 (s, 3H), 3.53 (s, 2H).
FI-MS (Turboscan): m/z (negative mode): 358.8
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 2.67 min (HPLC purity: 98.0%).

### Intermediate 5 : 1-biphenyl-4-yl-pyrazolidine-3,5-dione

To a solution of 1-(4-iodo-phenyl)-pyrazolidine-3,5-dione (Intermediate 1, 604 mg, 2.0 mmol), phenyl boronic acid (293 mg, 2.4 mmol) and MeOH (1 mL) in DMF (15 mL) was added an aqueous solution of Na₂CO₃ (2M, 1.2 mL) under inert atmosphere. The resulting mixture was degassed for 20 min and the tetrakis triphenylphosphine palladium (116 mg, 0.10 mmol) was added at once. After stirring at 110°C for 3 h under an inert atmosphere, acetic acid (1 mL) was added. The reaction mixture was filtered on a bed of celite and the collected solid washed with DMF. After evaporation of the solvents, a pale yellow powder (1.250 g) precipitated out after addition of Et₂O. Purification by preparative HPLC (starting at 20% ACN to 100% ACN in 60 min. in 0.1%TFA in H₂O) gave 75 mg of 1-biphenyl-4-yl-pyrazolidine-3,5-dione after freeze-drying (14 %).
¹H NMR (DMSO-d₆, 300 MHz) δ 11.2-11.8 (br s, 1H), 7.60-7.49 (m, 9H), 3.62 (s, 2H). FI-MS (APCI): m/z (negative mode): 250.8
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 4.22 min (HPLC purity: 98.0%).

### Example 1 : 4-(4-Iodo-benzylidene)-1-(4-iodo-phenyl)-pyrazolidine-3.5-dione

To a suspension of 1-(4-iodo-phenyl)-pyrazolidine-3,S-dione (Intermediate 1, 30 mg, 0.10 mmol) in EtOH (1.5 mL) were added 4-iodo-benzaldehyde (18 mg, 0.12 mmol) and one drop of pyridine under inert atmosphere. After stirring overnight at 70°C, the reaction mixture was cooled to rt and the orange solid precipitated out. Filtration on a fritté, washing with EtOH (2 x 1mL) and with Et₂O (3 x 1mL) and drying *in vacuo* at 40°C for 24h gave 34 mg of 4-(4-iodo-benzylidene)-1-(4-iodo-phenyl)-pyrazolidine-3,5-dione (87%).
¹H NMR (DMSO-d₆, 300 MHz) δ 11.0-12.0 (br s, 1H), 8.33 (m, 2H), 7.94 (dd, 2H, J1=1.5 Hz, J2=8.6 Hz), 7.90-7.75 (m, 3H), 7.58 (m, 2H)
FI-MS (APCI): m/z 514.8 (negative mode) and 516.0 (positive mode)

### Example 2 : 4-(Dimethyl-furan-2-ylmethylene)-1-(3-trifluoromethyl-phenyl)-pyrazolidine-3,5-dione

To a suspension of 1-(3-trifluoromethyl-phenyl)-pyrazolidine-3,5-dione (Intermediate 2, 24.4 mg, 0.10 mmol) in EtOH (1.5 mL) were added 4,5-dimethyl-furan-2-carbaldehyde (14.9 mg, 0.12 mmol) and one drop of pyridine under inert atmosphere. After stirring overnight at 70°C, the reaction mixture was cooled to rt and an orange solid precipitated out. Filtration on a fritté, washing with EtOH (2 x 1mL) and with Et₂O (3 x 1mL), and drying *in vacuo* at 40°C for 24h gave 11.9 mg of 4-(dimethyl-furan-2-ylmethylene)-1-(3-trifluoromethyl-phenyl)-pyrazolidine-3,5-dione (34%).
FI-MS (APCI): m/z 349.8 (negative mode) and 350.6 (positive mode)

### Example 3 : 1-Biphenyl-4-yl-4-furan-2-ylmethylene-pyrazolidine-3,5-dione

To a suspension of 1-biphenyl-4-yl-pyrazolidine-3,5-dione (Intermediate 5, 25.2 mg, 0.10 mmol) in EtOH (1.5 mL) were added furan-2-carbaldehyde (11.5 mg, 0.12 mmol) and one drop of pyridine under inert atmosphere. After stirring overnight at 70°C, the reaction mixture was cooled to rt and an orange solid precipitated out. Filtration on a fritté, washing with EtOH (2 x 1mL) and with Et₂O (3 x 1mL), and drying *in vacuo* at 40°C for 24h gave 18.4 mg of 1-biphenyl-4-yl-4-furan-2-ylmethylene-pyrazolidine-3,5-dione (56%).
¹H NMR (DMSO-d₆, 300 MHz) δ 11.32 (m, 1H), 8.49 (m, 1H), 8.27 (s, 1H), 7.78 (m, 4H), 7.68 (m, 3H), 7.47 (m, 2H), 7.36 (m, 1 H), 6.94 (s, 1H)
FI-MS (APCI): m/z 329.0 (negative mode) and 331.0 (positive mode)

### Example 4 : 4-(2-Hydroxy-benzylidene)-1-(4-iodo-phenyl)-pyrazolidine-3,5-dione

The same procedure as employed in the preparation of Example 1, but starting from 2-hydroxy-benzaldehyde, gave 24 mg of 4-(2-hydroxy-benzylidene)-1-(4-iodo-phenyl)-pyrazolidine-3,5-dione as an orange powder (71%).
¹H NMR (DMSO-d₆, 300 MHz) δ 10.12 (s, 1H), 8.78 (s, 1H), 8.27 (s, 1H), 7.97 (dd, 1H, J1=1.5Hz, J2=7.9Hz), 7.76 (m, 1H), 7.54-7.43 (m, 4H), 6.67 (m, 2H)
FI-MS (APCI): m/z (negative mode): 405.0
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 5.4 min (HPLC purity: 90.7%).

### Example 5 : 4-Furan-2-ylmethylene-1-(4-iodo-phenyl)-pyrazolidine-3,5-dione

The same procedure as employed in the preparation of Example 1 but starting from furan-2-carbaldehyde gave 51 mg of 4-furan-2-ylmethylene-1-(4-iodo-phenyl)-pyrazolidine-3,5-dione as a fine brown powder (73%).
FI-MS (APCI): m/z (negative mode): 378.8
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 4.98 min (HPLC purity: 94%).

### Example 6:4-(5-Bromo-2-hydroxy-benzylidene)-1-(4-iodo-phenyl)-pyrazolidine-3,5-dione

The same procedure as employed in the preparation of Example 1 but starting from 5-bromo-2-hydroxy-benzaldehyde gave 33 mg of 4-(5-bromo-2-hydroxy-benzylidene)-1-(4-iodo-phenyl)-pyrazolidine-3,5-dione as a yellow solid (81%).
¹H NMR (DMSO-d₆, 300 MHz) δ 10.12 (br s, 1H), 8.72 (s, 1H), 8.26 (m, 2H,), 7.90 (m, 1H), 7.48 (m, 3H), 6.66 (m, 2H)
FI-MS (APCI): m/z (negative mode): 482.6
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 6.32 min (HPLC purity: 98.3%).

### Example 7 : 4-(3-Iodo-benzylidene)-1-(4-iodo-phenyl)-pyrazolidine-3,5-dione

The same procedure as employed in the preparation of Example 1 but starting from 3-iodobenzaldehyde gave 32 mg of 4-(3-iodo-benzylidene)-1-(4-iodo-phenyl)-pyrazolidine-3,5-dione as a fine brown powder (75%).
¹H NMR (DMSO-d₆, 300 MHz) δ 11.51 (br s, 1H), 9.13 (m, 0.5H iso A), 8.97 (m, 0.5H iso B), 8.42 (d, 1H, J=7.9Hz), 7.96 (d, 1H, J=7.1Hz), 7.79 (m, 3H), 7.58 (m, 2H), 7.35 (t, 1H, J=7.9Hz); FI-MS (APCI): m/z (negative mode): 514.6
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 6.67 and 6.82 min (HPLC purity: 95.1%).

### Example 8 : 4-Benzylidene-1-(4-iodo-phenyl)pyrazolidine-3,5-dione

The same procedure as employed in the preparation of Example 1 but starting from benzaldehyde gave 8.8 mg of 4-benzylidene-1-(4-iodo-phenyl)-pyrazolidine-3,5-dione as a red powder (28%).
FI-MS (APCI): m/z (positive mode): 391.0 and m/z (negative mode): 388.8
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 4.59 and 4.70 min (HPLC purity: 89.3%).

### Example 9 : 4-(4-Dimethylamino-benzylidene)-1-('4-iodo-phenyl)-pyrazolidine-3,5-dione

The same procedure as employed in the preparation of Example 1 but starting from 4-dimethylamino-benzaldehyde gave 28.3 mg of 4-(4-dimethylamino-benzylidene)-1-(4-iodo-phenyl)-pyrazolidine-3,5-dione as a red powder (70%).
FI-MS (Turboscan): m/z (negative mode): 431.8
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 4.5 min (HPLC purity: 84.9%).

### Example 10 : 4-[1-(4-Iodo-phenyl)-3,5-dioxo-pyrazolidin-4-ylidenemethyl]-benzoic acid methyl ester

The same procedure as employed in the preparation of Example 1 but starting from 4-formyl-benzoic acid methyl ester gave 26 mg of 4-[1-(4-iodo-phenyl)-3,5-dioxo-pyrazolidin-4-ylidenemethyl]-benzoic acid methyl ester as a brown fine powder (76%). ¹H NMR (DMSO-d₆, 300 MHz) δ 11.52 (br s, 1H), 8.60 (m, 2H), 8.06 (d, 2H, J=8.3Hz), 7.95 (m, 1H), 7.80 (m, 2H), 7.60 (m, 2H), 3.89 (s, 3H)
FI-MS (APCI): m/z (negative mode): 447.0
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 5.91 min (HPLC purity: 91.1 %).

### Example 11 : 1-(4-Iodo-phenyl)-4-thiophen-2-ylmethylene-pyrazolidine-3,5-dione

The same procedure as employed in the preparation of Example 1 but starting from thiophene-2-carbaldehyde gave 17.4 mg of 1-(4-iodo-phenyl)-4-thiophen-2-ylmethylene-pyrazolidine-3,5-dione as an orange solid (49%).
¹H NMR (DMSO-d₆, 300 MHz) δ 11.15 (br s, 1H), 8.4-8.1 (m, 3H), 7.78 (m, 2H), 7.55 (m, 2H), 7.36 (t, 1H)
FI-MS (Turboscan): m/z (negative mode): 394.8
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 5.38 and 5.46 min (HPLC purity: 88.6%).

### Example 12 : 4-[1-(4-Iodo-phenyl)-3,5-dioxo-pyrazolidin-4-ylidenemethyl]-benzoic acid

The same procedure as employed in the preparation of Example 1 but starting from 4-formyl-benzoic acid gave 33 mg of 4-[1-(4-iodo-phenyl)-3,5-dioxo-pyrazolidin-4-ylidenemethyl]-benzoic acid as a brown fine powder (86%).
¹H NMR (DMSO-d₆, 300 MHz) δ 13.1 (bs, 1H), 8.57 (d, 2H, J=8.3 Hz), 8.05 (d, 2H, J=8.4 Hz), 7.94 (m, 1H), 7.79 (m, 2H), 7.59 (t, 2H, J=9.0 Hz)
FI-MS (APCI): m/z (negative mode): 433.0
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 4.93 and 5.11 min (HPLC purity: 79.6%).

### Example 13 : {4-[1-(4-Iodo-phenyl)-3,5-dioxo-pyrazolidin-4-ylidenemethyl]-phenoxy}-acetic acid

The same procedure as employed in the preparation of Example 1 but starting from (4-formyl-phenoxy)-acetic acid gave 29 mg of {4-[1-(4-iodo-phenyl)-3,5-dioxo-pyrazolidin-4-ylidenemethyl]-phenoxy}-acetic acid as an orange powder (74%).
FI-MS (APCI): m/z (positive mode): 465.0 and m/z (negative mode): 462.8
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 5.02 min (HPLC purity: 97.3%).

### Example 14 : (E)-3-{4-[1-(4-Iodo-phenyl)-3,5-dioxo-pyrazolidin-4-ylidenemethyl]-phenyl}-acrylic acid

The same procedure as employed in the preparation of Example 1 but starting from (E)-3-(4-formyl-phenyl)-acrylic acid gave 27 mg of (E)-3-{4-[1-(4-iodo-phenyl)-3,5-dioxo-pyrazolidin-4-ylidenemethyl]-phenyl}-acrylic acid as a brown powder (67%).
FI-MS (APCI): m/z (negative mode): 458.8
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 5.13 and 5.30 min (HPLC purity: 92.3%).

### Example 15 : 2-Hydroxy-5-[1-(4-iodo-phenyl)-3,5-dioxo-pYrazolidin-4-ylidenemethyl]-benzoic acid

The same procedure as employed in the preparation of Example 1 but starting from 5-formyl-2-hydroxy-benzoic acid gave 25 mg of 2-hydroxy-5-[1-(4-iodo-phenyl)-3,5-dioxo-pyrazolidin-4-ylidenemethyl]-benzoic acid as an orange powder (64%).
FI-MS (APCI): m/z (positive mode): 446.8 and m/z (negative mode): 448.1
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 5.19 and 5.27 min (HPLC purity: 97.1 %).

### Example 16 : 3-[1-(4-Iodo-phenyl)-3,5-dioxo-pyrazolidin-4-ylidenemethyl]-benzoic acid

The same procedure as employed in the preparation of Example 1 but starting from 3-formyl-benzoic acid gave 21 mg of 3-[1-(4-iodo-phenyl)-3,5-dioxo-pyrazolidin-4-ylidenemethyl]-benzoic acid as an orange powder (58%).
FI-MS (APCI): m/z (positive mode): 433.0 and m/z (negative mode): 435.1
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 4.99 and 5.09 min (HPLC purity: 93.8%).

### Example 17 : (E)-3-{4-[3,5-Dioxo-1-(3-trifluoromethyl-phenyl)-pyrazolidin-4-ylidene-methyl]-phenyl}-acrylic acid

The same procedure as employed in the preparation of Example 2 but starting from (E)-3-(4-formyl-phenyl)-acrylic acid gave 13 mg of (E)-3-{4-[3,5-dioxo-1-(3-trifluoromethylphenyl)-pyrazolidin-4-ylidenemethyl]-phenyl}-acrylic acid as an orange solid (45%).
FI-MS (APCI): m/z (negative mode): 400.8
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 5.25 and 5.42 min (HPLC purity: 98.6%).

### Example 18 : {2-[1-(4-Iodo phenyl)-3,5-dioxo-pyrazolidin-4-ylidenemethyl]-phenoxy}-acetic acid

The same procedure as employed in the preparation of Example 1 but starting from (2-formyl-phenoxy)-acetic acid gave 25 mg of {2-[1-(4-iodo-phenyl)-3,5-dioxo-pyrazolidin-4-ylidenemethyl]-phenoxy}-acetic acid as an orange solid (62%).
FI-MS (APCI): m/z (positive mode): 465.0
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 5.03 and 5.33 min (HPLC purity: 90.7%).

### Example 19 : {4-[3.5-Dioxo-1-(3-trifluoromethyl-phenyl)-pyrazolidin-4-ylidenemethyl]-phenoxy}-acetic acid

The same procedure as employed in the preparation of Example 2 but starting from (4-formyl-phenoxy)-acetic acid gave 11 mg of {4-[3,5-dioxo-1-(3-trifluoromethyl-phenyl)-pyrazolidin-4-ylidenemethyl]-phenoxy}-acetic acid as an orange powder (37%).
FI-MS (APCI): m/z (positive mode): 407.0 and m/z (negative mode): 405.0
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 5.03 and 5.15 min (HPLC purity: 97.3%).

### Example 20 : 5-[3,5-Dioxo-1-(3-trifluoromethyl-phenyl)-pyrazolidin-4-ylidenemethyl]-2-hydroxy-benzoic acid

The same procedure as employed in the preparation of Example 2 but starting from 5-formyl-2-hydroxy-benzoic acid gave 12 mg of 5-[3,5-dioxo-1-(3-trifluoromethyl-phenyl)-pyrazolidin-4-ylidenemethyl]-2-hydroxy-benzoic acid as an orange powder (44%).
FI-MS (APCI): m/z (positive mode): 392.8 and m/z (negative mode): 390.8
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 5.30 and 5.39 min (HPLC purity: 92.5%).

### Example 21 : {2-[3,5-Dioxo-1-(3-trifluoromethyl-phenyl)-pyrazolidin-4-ylidenemethyl]-phenoxy}-acetic acid

The same procedure as employed in the preparation of Example 2 but starting from (2-formyl-phenoxy)-acetic acid gave 11 mg of {2-[3,5-dioxo-1-(3-trifluoromethyl-phenyl)-pyrazolidin-4-ylidenemethyl]-phenoxy}-acetic acid as an orange powder (40%).
FI-MS (APCI): m/z (positive mode): 407.0 and m/z (negative mode): 404.8
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 5.13 and 5.41 min (HPLC purity: 92.6%).

### Example 22 : Acetic acid 5-[1-(4-iodo-phenyl)-3,5-dioxo-pyrazolidin-4-ylidenemethyl]-furan-2-ylmethyl ester

The same procedure as employed in the preparation of Example 1 but starting from acetic acid 5-formyl-furan-2-ylmethyl ester gave 14.6 mg of acetic acid 5-[1-(4-iodo-phenyl)-3,5-dioxo-pyrazolidin-4-ylidenemethyl]-furan-2-ylmethyl ester as an orange powder (38%). FI-MS (APCI): m/z (positive mode): 453.0 and m/z (negative mode): 450.8
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 5.46 and 5.50 min (HPLC purity: 81.8%).

### Example 23 : 4-(4-Bromo-furan-2-ylmethylene)-1-(4-iodo-phenyl)-pyrazolidine-3,5-dione

The same procedure as employed in the preparation of Example 1 but starting from 4-bromo-furan-2-carbaldehyde gave 18.2 mg of 4-(4-bromo-furan-2-ylmetbylene)-1-(4-iodophenyl)-pyrazolidine-3,5-dione as a camel powder (46%).
¹H NMR (DMSO-d₆, 300 MHz) δ 8.49 (m, 2H), 7.78 (m, 2H), 7.57 (m, 4H)
FI-MS (APCI): m/z (negative mode): 458.6
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 6.00 and 6.30 min (HPLC purity: 81.7%).

### Example 24 : 1-(4-Iodo-phenyl)-4-(5-methyl-furan-2-ylmethylene)-pyrazolidine-3,5-dione

The same procedure as employed in the preparation of Example 1 but starting from 5-methyl-furan-2-carbaldehyde gave 24.2 mg of 1-(4-iodo-phenyl)-4-(5-methyl-furan-2-ylmethylene)-pyrazolidine-3,5-dione as an orange powder (69%).
¹H NMR (DMSO-d₆, 300 MHz) δ 11.0 (bs, 1H), 8.49 (m, 1H), 7.76 (m, 2H), 7.55 (m, 3H), 6.64 (d, 1H, J=3.8Hz), 2.47 (s, 3H)
FI-MS (APCI): m/z (positive mode): 394.8 and m/z (negative mode): 392.8
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 5.56 and 5.61 min (HPLC purity: 82.3%).

### Example 25 : 4-(5-Bromo-furan-2-ylmethylene)-1-(4-iodo-phenyl)-pyrazolidine-3,5-dione

The same procedure as employed in the preparation of Example 1 but starting from 5-bromo-furan-2-carbaldehyde gave 20.9 mg of 4-(5-bromo-furan-2-ylmethylene)-1-(4-iodophenyl)-pyrazolidine-3,5-dione as a brown orange powder (58%).
¹H NMR (DMSO-d₆, 300 MHz) δ 11.30 (br s, 1H), 8.40 (m, 1H), 7.79 (dd, 2H, J1=3.0Hz, J2=9.0Hz), 7.57 (m, 3H), 7.06 (d, 1H, J1=3.8Hz)
FI-MS (APCI): m/z (negative mode): 456.6
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 6.00 and 6.08 min (HPLC purity: 85.1%).

### Example 26 : 1-(4-Iodo-phenyl)-4-[5-(2-methoxy-phenyl)-thiophen-2-ylmethylene]-pyrazolidine-3,5-dione

### Step 1 : Preparation of 5-(2-methoxy-phenyl)-thiophene-2-carbaldehyde

To a mixture of 5-bromothiophene-2-carboxaldehyde (40 g, 210 mmol), 2-methoxy-benzeneboronic acid (38 g, 250 mmol) and sodium carbonate (80 g) in toluene (700 mL) and water (400 mL) was added Pd(PPh₃)₄ (12 g, 10 mmol) with stirring and refluxed under nitrogen atmosphere for 5 h. The toluene layer was separated, washed with water and concentrated. The crude was purified by column chromatography over silica gel (PetEther/CH₂Cl₂) to give 26 g of 5-(2-methoxy-phenyl)-thiophene-2-carbaldehyde as a white powder (57 %).
¹H NMR (CDCl₃, 300 MHz) δ 9.95 (s, 1H), 7.99 (d, 1H, J=4.1 Hz), 7.89 (d, 1H, J=7.5 Hz), 7.81 (d, 1H, J=4.1 Hz), 7.41 (dd, 1H, J1=J2=7.6 Hz), 7.20 (d, 1H, J=8.3 Hz), 7.07 (dd, 1H, J1=J2=7.9 Hz), 4.05 (s, 3H).
HPLC (C8 Waters Symmetry Column, 4.6 x 50 mm², UV detection at 254 nm, 2 mL/min, 10 min gradient from 0.1 % TFA in H₂O to 0.07 % TFA in ACN): retention time of 6.72 min (HPLC purity: 97.5%).

### Step 2: Preparation of 1-(4-Iodo-phenyl)-4-[5-(2-methoxy-phenyl)-thiophen-2-yl methylene]-pyrazolidine-3,5-dione

The same procedure as employed in the preparation of Example 1 but starting from 5-(2-methoxy-phenyl)-thiophene-2-carbaldehyde (obtained from Step 1) gave 32.1 mg of 1-(4-iodo-phenyl)-4-[5-(2-methoxy-phenyl)-thiophen-2-ylmethylene]-pyrazolidine-3,5-dione as an orange powder (72%).
¹H NMR (DMSO-d₆, 300 MHz) δ 10.94 (br s, 1H), 8.26 (m, 1H), 8.02 (m, 1H), 7.83 (m, 2H), 7.72 (m, 2H), 7.51 (m, 2H), 7.39 (m, 1H), 7.17 (m, 1H), 7.04 (m, 1H), 3.98 (s, 3H) FI-MS (APCI): m/z (positive mode): 503.0 and m/z (negative mode): 500.8
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 6.63 min (HPLC purity: 95.7%).

### Example 27 : 1-(4-Iodo-phenyl)-4-thiophen-3-ylmethylene-pyrazolidine-3,5-dione

The same procedure as employed in the preparation of Example 1 but starting from thiophene-3-carbaldehyde gave 9.7 mg of 1-(4-iodo-phenyl)-4-thiophen-3-ylmethylene-pyrazolidine-3,5-dione as an orange solid (37%).
FI-MS (Turboscan): m/z (negative mode): 394.8
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 5.59 and 5.81 min (HPLC purity: 98.1%).

### Example 28 : 1-(4-Iodo-phenyl)-4-(5-phenyl-thiophen-2-ylmethylene)-pyrazolidine-3,5-dione

The same procedure as employed in the preparation of Example 1 but starting from 5-phenyl-thiophene-2-carbaldehyde gave 50 mg of 1-(4-iodo-phenyl)-4-(5-phenyl-thiophen-2-ylmethylene)-pyrazolidine-3,5-dione as a brown solid (56%).
¹H NMR (DMSO-d₆, 300 MHz) δ 11.17 (br s, 1H), 8.25 (m, 1H), 8.10 (m, 1H), 7.81 (m, 5H), 7.52 (m, 5H)
FI-MS (Turboscan): m/z (negative mode): 471.0
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 6.60 and 6.75 min (HPLC purity: 87.2%).

### Example 29 : 4-Furan-2-ylmethylene-1-(3-trifluoromethyl-phenyl)-pyrazolidine-3,5-dione

The same procedure as employed in the preparation of Example 2 but starting from furan-2-carbaldehyde gave 36 mg of 4-furan-2-ylmethylene-1-(3-trifluoromethyl-phenyl)-pyrazolidine-3,5-dione as an orange solid (63%).
FI-MS (Turboscan): m/z (negative mode): 321.0
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 5.41 and 5.51 min (HPLC purity: 96.2%).

### Example 30 : 4-(5-Methyl-furan-2-ylmethylene)-1-(3-trifluoromethyl-phenyl)-pyrazolidine-3,5-dione

The same procedure as employed in the preparation of Example 2 but starting from 5-methyl-furan-2-carbaldehyde gave 34 mg of 4-(5-methyl-furan-2-ylmethylene)-1-(3-trifluoromethyl-phenyl)-pyrazolidine-3,5-dione as a pale orange solid (86%).
FI-MS (Turboscan): m/z (negative mode): 335.0
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 5.72 min (HPLC purity: 88.3%).

### Example 31 : 4-Thiophen-2-ylmethylene-1-(3-trifluoromethyl-phenyl)-pyrazolidine-3,5-dione

The same procedure as employed in the preparation of Example 2 but starting from thiophene-2-carbaldehyde gave 24 mg of 4-thiophen-2-ylmethylene-1-(3-trifluoromethylphenyl)-pyrazolidine-3,5-dione as a red orange solid (86%).
FI-MS (Turboscan): m/z (negative mode): 337.0
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 5.57 and 5.64 min (HPLC purity: 96.5%).

### Example 32 : 4-(4-Bromo-furan-2-ylmethylene)-1-(3-trifluoromethyl-phenyl)-pyrazolidine-3.5-dione

The same procedure as employed in the preparation of Example 2 but starting from 4-bromo-furan-2-carbaldehyde gave 21 mg of 4-(4-bromo-furan-2-ylmethylene)-1-(3-trifluoromethyl-phenyl)-pyrazolidine-3,5-dione as a brown solid (65%).
¹H NMR (DMSO-d₆, 300 MHz) δ 11.5 (br s, 1H), 8.53 (m, 2H), 8.20-8.00 (m, 2H), 7.72-7.50 (m, 3H)
FI-MS (Turboscan): m/z (negative mode): 398.6
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 6.12 and 6.39 min (HPLC purity: 99.3%).

### Example 33 : Acetic acid 5-[3,5-dioxo-1-(3-trifluoromethyl-phenyl)-pyrazolidin-4-ylidenemethyl]-furan-2-ylmethyl ester

The same procedure as employed in the preparation of Example 2 but starting from acetic acid 5-formyl-furan-2-ylmethyl ester gave 53 mg of acetic acid 5-[3,5-dioxo-1-(3-trifluoromethyl-phenyl)-pyrazolidin-4-ylidenemethyl]-furan-2-ylmethyl ester as an orange solid (76%).
FI-MS (Turboscan): m/z (negative mode): 393.0
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 5.62 min (HPLC purity: 88.6%).

### Example 34 : 1-(3-Chloro-4-methyl-phenyl)-4-furan-2-ylmethylene-pyrazolidine-3,5-dione

To a suspension of 1-(3-chloro-4-methyl-phenyl)-pyrazolidine-3,5-dione (Intermediate 3, 22.4 mg, 0.10 mmol) in EtOH (1.5 mL) were added furan-2-carbaldehyde (11.5 mg, 0.12 mmol) and one drop of pyridine under inert atmosphere. After stirring overnight at 70°C, the reaction mixture was cooled to rt and the orange solid precipitated out. Filtration on a fritté, washing with EtOH (2 x 1mL) and with Et₂O (3 x 1mL) and drying *in vacuo* at 40°C for 24h gave 26 mg of 1-(3-chloro-4-methyl-phenyl)-4-furan-2-ylmethylene-pyrazolidine-3,5-dione as an orange solid (51%).
¹H NMR (DMSO-d₆, 300 MHz) δ 11.26 (br s, 1H), 8.51 (m, 1H), 8.27 (s, 1H), 8.85 (m, 1H), 7.65-7.54 (m, 2H), 7.42 (m, 1H), 6.93 (m, 1H), 2.32 (s, 3H)
FI-MS (Turboscan): m/z (negative mode): 300.8
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 5.28 and 5.39 min (HPLC purity: 95.6%).

### Example 35 : 4-(7-Bromo-8-hydroxy-quinolin-5-ylmethylene)-1-(4-iodo-phenyl)-pyrazolidine-3,5-dione

The same procedure as employed in the preparation of Example 1 but starting from 7-bromo-8-hydroxy-5-quinolinecarbaldehyde (prepared according to the procedure of Mongin, F. et al Tetrahedron Lett. 1995, 36(46), 8415-18) gave 27.4 mg of 4-(7-bromo-8-hydroxy-quinolin-5-ylmethylene)-1-(4-iodo-phenyl)-pyrazolidine-3,5-dione as a brown fine powder (60%).
FI-MS (APCI): m/z (positive mode): 535.8 and m/z (negative mode): 534.0
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 4.75 min (HPLC purity: 88%).

### Example 36 : 1-(4-Iodo-phenyl)-4-[5-(4-nitro-phenyl)-furan-2-ylmethylene]-pyrazolidine-3,5-dione

The same procedure as employed in the preparation of Example 1 but starting from 5-(4-nitrophenyl)-furan-2-carbaldehyde gave 14.1 mg of 1-(4-iodo-phenyl)-4-[5-(4-nitrophenyl)-furan-2-ylmethylene]-pyrazolidine-3,5-dione as a red solid (38%).
¹H NMR (DMSO-d₆, 300 MHz) δ 11.33 (br s, 1H), 8.49 (d, 0.5H, J=3.7 Hz), 8.32-8.42 (m, 2.5H), 8.20 (m, 2H), 7.79 (m, 2H), 7.70 (m, 2H), 7.66-7.53 (m, 2H)
FI-MS (APCI): m/z (positive mode): 502.0 and m/z (negative mode): 499.8
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 6.71 min (HPLC purity: 98.8%).

### Example 37 : 1-(4-Iodo-phenyl)-4-[5-(2-nitro-phenyl)-furan-2-ylmethylene]-pyrazolidine-3,5-dione

The same procedure as employed in the preparation of Example 1 but starting from 5-(2-nitrophenyl)-furan-2-carbaldehyde gave 5.3 mg of 1-(4-iodo-phenyl)-4-[5-(2-nitro-phenyl)-furan-2-ylmethylene]-pyrazolidine-3,5-dione as a brown solid (21%).
FI-MS (APCI): m/z (positive mode): 502.0 and m/z (negative mode): 499.8
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 6.49 min (HPLC purity: 93.8%).

### Example 38 : 4-(5-Hydroxymethyl-furan-2-ylmethylene)-1-(4-iodo-phenyl)-pyrazolidine-3,5-dione

The same procedure as employed in the preparation of Example 1 but starting from 5-hydroxymethyl-furan-2-carbaldehyde gave 5.1 mg of 4-(5-hydroxymethyl-furan-2-ylmethylene)-1-(4-iodo-phenyl)-pyrazolidine-3,5-dione as a red solid (25%).
FI-MS (APCI): m/z (positive mode): 410.8 and m/z (negative mode): 408.8
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 4.55 min (HPLC purity: 96.8%).

### Example 39 : 4-((E)-3-Furan-2-yl-allylidene)-1-(4-iodo-phenyl)-pyrazolidine-3.5-dione

The same procedure as employed in the preparation of Example 1 but starting from (E)-3-furan-2-yl-propenal gave 6.1 mg of 4-((E)-3-furan-2-yl-allylidene)-1-(4-iodo-phenyl)-pyrazolidine-3,5-dione as a brown solid (27%).
¹H NMR (DMSO-d₆, 300 MHz) δ 12.39 (br s, 0.4H, isoA), 11.12 (br s, 0.6H, isoB), 8.11-7.65 (m, 5H), 7.65-7.48 (m, 3H), 7.05 (d, 1H, J=3.0 Hz), 6.73 (m, 1H)
FI-MS (APCI): m/z (positive mode): 406.6 and m/z (negative mode): 404.8
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 5.79 min (HPLC purity: 98.8%).

### Example 40 : 4-Furan-3-ylmethylene-1-(4-iodo-phenyl)-pyrazolidine-3,5-dione

The same procedure as employed in the preparation of Example 1 but starting from furan-3-carbaldehyde gave 10.5 mg of 4-furan-3-ylmethylene-1-(4-iodo-phenyl)-pyrazolidine-3,5-dione as an orange solid (41 %).
FI-MS (APCI): m/z (positive mode): 380.8 and m/z (negative mode): 378.6
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 5.39 and 5.61 min (HPLC purity: 90.5%).

### Example 41:1-(4-Iodo-phenyl)-4-(5-nitro-thiophen-2-ylmethylene)-pyrazolidine-3,5-dione

The same procedure as employed in the preparation of Example 1 but starting from 5-nitrothiophene-2-carbaldehyde gave 10 mg of 1-(4-iodo-phenyl)-4-(5-nitro-thiophen-2-ylmethylene)-pyrazolidine-3,5-dione as a brown solid (34%).
¹H NMR (DMSO-d₆, 300 MHz) δ 11.68 (br s, 1H), 8.30-8.12 (m, 3H), 7.85-7.75 (m, 2H), 7.69-7.48 (m, 2H)
FI-MS (APCI): m/z (positive mode): 441.8 and m/z (negative mode): 439.6
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 5.92 and 6.22 min (HPLC purity: 98.9%).

### Example 42 : 4-(5-Hydroxymethyl-furan-2-ylmethylene)-1-(3-trifluoromethyl-phenyl)-pyrazolidine-3,5-dione

The same procedure as employed in the preparation of Example 2 but starting from 5-hydroxymethyl-furan-2-carbaldehyde gave 7.5 mg of 4-(5-hydroxymethyl-furan-2-ylmethylene)-1-(3-trifluoromethyl-phenyl)-pyrazolidine-3,5-dione as an orange solid (36%).
¹H NMR (DMSO-d₆, 300 MHz) δ 11.50 (br s, 1H), 8.60-8.35 (m, 1H), 8.19 (s, 1H), 8.13-7.98 (m, 1H), 7.74-7.65 (m, 1H), 7.63-7.50 (m, 2H), 6.79 (d, 1H, J=3.4Hz), 5.64 (m, 1H), 4.58 (d, 2H, J=5.3Hz)
FI-MS (APCI): m/z (positive mode): 353.0 and m/z (negative mode): 350.8
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 4.67 min (HPLC purity: 97.5%).

### Example 43 : 4-Furan-3-ylmethylene-1-(3-trifluoromethyl-phenyl)-pyrazolidine-3,5-dione

The same procedure as employed in the preparation of Example 2 but starting from furan-3-carbaldehyde gave 3.2 mg of 4-furan-3-ylmethylene-1-(3-trifluoromethyl-phenyl)-pyrazolidine-3,5-dione as an orange solid (25%).
FI-MS (APCI): m/z (positive mode): 323.0 and m/z (negative mode): 321.8
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 5.60 and 5.79 min (HPLC purity: 96.8%).

### Example 44 : 4-(5-Nitro-thiophen-2-ylmethylene)-1-(3-trifluoromethyl-phenyl)-pyrazolidine-3,5-dione

The same procedure as employed in the preparation of Example 2 but starting from 5-nitrothiophene-2-carbaldehyde gave 7.4 mg of 4-(5-nitro-thiophen-2-ylmethylene)-1-(3-trifluoromethyl-phenyl)-pyrazolidine-3,5-dione as a brown solid (32%).
FI-MS (APCI): m/z (positive mode): 383.8 and m/z (negative mode): 381.8
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 6.06 and 6.30 min (HPLC purity: 98.3%).

### Example 45 : 1-(4-Iodo-phenyl)-4-(5-nitro-furan-2-ylmethylene)-pyrazolidine-3,5-dione

The same procedure as employed in the preparation of Example 1 but starting from 5-nitro-furan-2-carbaldehyde gave 16.6 mg of 1-(4-iodo-phenyl)-4-(5-nitro-furan-2-ylmethylene)-pyrazolidine-3,5-dione as a dark brown powder (51%).
FI-MS (Turbo Scan): m/z (negative mode): 423.8
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 5.75 min (HPLC purity: 97.6%).

### Example 46 : 1-(4-Iodo-phenyl)-4-[5-(3-nitro-phenyl)-furan-2-ylmethylene]-pyrazolidine-3,5-dione

The same procedure as employed in the preparation of Example 1 but starting from 5-(3-nitro-phenyl)-furan-2-carbaldehyde gave 19.5 mg of 1-(4-iodo-phenyl)-4-[5-(3-nitrophenyl)-furan-2-ylmethylene]-pyrazolidine-3,5-dione as a brown powder (50%).
¹H NMR (DMSO-d₆, 300 MHz) δ 11.22 (br s, 1H), 8.75 (d, 1H, J=10.2Hz), 8.48-8.36 (m, 2H), 8.25 (m, 1H), 7.83-7.68 (m, 5H), 7.58 (m, 2H)
FI-MS (APCI): m/z (positive mode): 502.0 and m/z (negative mode): 499.8
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 6.56 min (HPLC purity: 93.7%).

### Example 47 : 4-(4,5-Dimethyl-furan-2-ylmethylene)-1-(4-iodo-phenyl)-pyrazolidine-3,5-dione

The same procedure as employed in the preparation of Example 1 but starting from 4,5-dimethyl-furan-2-carbaldehyde gave 14.6 mg of 4-(4,5-dimethyl-furan-2-ylmethylene)-1-(4-iodo-phenyl)-pyrazolidine-3,5-dione as an orange-red powder (47%).
¹H NMR (DMSO-d₆, 300 MHz) δ 8.42 (s, 0.6H iso A), 8.33 (s, 0.4H iso B), 7.45-7.60 (m, 3H), 7.52 (m, 2H), 2.38 (s, 3H), 2.04 (s, 3H)
FI-MS (APCI): m/z (positive mode): 408.4 and m/z (negative mode): 406.4
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 5.87 min (HPLC purity: 90.5%).

### Example 48 : 5-[1-(4-Iodo-phenyl)-3,5-dioxo-pyrazolidin-4- ylidenemethyl]-furan-2-sulfonic acid

The same procedure as employed in the preparation of Example 1 but starting from 5-Formyl-furan-2-sulfonic acid gave 5.3 mg of 5-[1-(4-iodo-phenyl)-3,5-dioxo-pyrazolidin-4-ylidenemethyl]-furan-2-sulfonic acid as an orange powder (22%).
¹H NMR (DMSO-d₆, 300 MHz) δ 11.26 (br s, 1H), 8.47 (m, 1H), 7.79 (dd, 2H, J1=3.1 Hz, J2=9.0 Hz), 7.40-7.70 (m, 4H)
FI-MS (Turboscan): m/z (negative mode): 458.4
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 4.07 and 4.18 min (HPLC purity: 92.6%).

### Example 49 : 4-[1-(4-Iodo-phenyl)-3,5-dioxo-pyrazolidin-4-ylidenemethyl]-benzonitrile

The same procedure as employed in the preparation of Example 1 but starting from 4-formyl-benzonitrile gave 16.4 mg of 4-[1-(4-iodo-phenyl)-3,5-dioxo-pyrazolidin-4-ylidenemethyl]-benzonitrile as an orange powder (82%).
¹H NMR (DMSO-d₆, 300 MHz) δ 11.69 (br s, 1H), 8.60 (m, 2H), 8.03-7.92 (m, 3H), 7.79 (m, 2H), 7.59 (m, 2H)
FI-MS (APCI): m/z (negative mode): 413.8
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 5.77 min (HPLC purity: 95.6%).

### Example 50 : 1-(4-Iodo-phenyl)-4-(3-methyl-thiophen-2-ylmethylene)-pyrazolidine-3,5-dione

The same procedure as employed in the preparation of Example 1 but starting from 3-methyl-thiophene-2-carbaldehyde gave 20.8 mg of 1-(4-iodo-phenyl)-4-(3-methyl-thiophen-2-ylmethylene)-pyrazolidine-3,5-dione as an orange powder (75%). ¹H NMR (DMSO-d₆, 300 MHz) δ 11.08 (br s, 1H), 8.19 (d, 1H, J=4.9Hz), 8.02 (s, 1H), 7.79 (m, 2H), 7.54 (m, 2H), 7.23 (d, 1H, J=4.9Hz), 2.54 (s, 3H)
FI-MS (Turboscan): m/z (negative mode): 408.6
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 5.59 min (HPLC purity: 94.9%).

### Example 51 : 4-(4-Bromo-thiophen-2-ylmethylene)-1-(4-iodo-phenyl)-pyrazolidine-3,5-dione

The same procedure as employed in the preparation of Example 1 but starting from 4-bromo-thiophene-2-carbaldehyde gave 16.7 mg of 4-(4-bromo-thiophen-2-ylmethylene)-1-(4-iodo-phenyl)-pyrazolidine-3,5-dione as an orange powder (61%).
¹H NMR (DMSO-d₆, 300 MHz) δ 11.34 (br s, 1H), 8.31 (m, 2H), 8.12 (m, 1H), 7.79 (m, 2H), 7.55 (m, 2H)
FI-MS (Turboscan): m/z (negative mode): 472.8
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 6.3 min (HPLC purity: 68.8%).

### Example 52 : 1-(4-Iodo-phenyl)-4-(5-methyl-thiophen-2-ylmethylene)-pyrazolidine-3,5-dione

The same procedure as employed in the preparation of Example 1 but starting from 5-methyl-thiophene-2-carbaldehyde gave 19.7 mg of 1-(4-iodo-phenyl)-4-(5-methyl-thiophen-2-ylmethylene)-pyrazolidine-3,5-dione as a dark red powder (71%).
FI-MS (Turboscan): m/z (negative mode): 408.8
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 5.64 min (HPLC purity: 84.8%).

### Example 53 : 4-Benzo[b]thiophen-2-ylmethylene-1-(4-iodo-phenyl)-pyrazolidine-3,5-dione

The same procedure as employed in the preparation of Example 1 but starting from benzo[b]thiophene-2-carbaldehyde gave 23.3 mg of 4-benzo[b]thiophen-2-ylmethylene-1-(4-iodo-phenyl)-pyrazolidine-3,5-dione as a red solid (63%).
FI-MS (APCI): m/z (positive mode): 447.0 and m/z (negative mode): 444.8
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 6.44 min (HPLC purity: 84.9%).

### Example 54 : 1-(4-Iodo-phenyl)-4-(4-nitro-thiophen-2-ylmethylene)-pyrazolidine-3,5-dione

The same procedure as employed in the preparation of Example 1 but starting from 4-nitrothiophene-2-carbaldehyde gave 27.9 mg of 1-(4-iodo-phenyl)-4-(4-nitro-thiophen-2-ylmethylene)-pyrazolidine-3,5-dione as a red solid (74%).
FI-MS (APCI): m/z (negative mode): 439.8
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 5.68 and 5.79 min (HPLC purity: 84.3%).

### Example 55 : 5[1-(4-Iodo-phenyl)-3,5-dioxo-pyrazolidin-4-ylidenemethyl]-thiophene-3-carboxylic acid

The same procedure as employed in the preparation of Example 1 but starting from 5-formyl-thiophene-3-carboxylic acid gave 19.7 mg of 5-[1-(4-iodo-phenyl)-3,5-dioxo-pyrazolidin-4-ylidenemethyl]-thiophene-3-carboxylic acid as a brown solid (56%).
FI-MS (APCI): m/z (negative mode): 440.6
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 4.79 min (HPLC purity: 93.9%).

### Example 56 : 4-[1-(4-Iodo-phenyl)-3,5-dioxo-pyrazolidin-4-ylidenemethyl]-thiophene-3-carboxylic acid

The same procedure as employed in the preparation of Example 1 but starting from 4-formyl-thiophene-3-carboxylic acid gave 3.5 mg of 4-[1-(4-iodo-phenyl)-3,5-dioxo-pyrazolidin-4-ylidenemethyl]-thiophene-3-carboxylic acid as an orange solid (19%). FI-MS (APCI): m/z (positive mode): 441.0
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 5.13 min (HPLC purity: 97.5%).

### Example 57 : 2-[3,5-Dioxo-1-(3-trifluoromethyl-phenyl)-pyrazolidin-4-ylidenemethyl]-thiophene-3-carboxylic acid

The same procedure as employed in the preparation of Example 2 but starting from 2-formyl-thiophene-3-carboxylic acid gave 11.9 mg of 2-[3,5-dioxo-1-(3-trifluoromethylphenyl)-pyrazolidin-4-ylidenemethyl]-thiophene-3-carboxylic acid as a brown solid (44%). FI-MS (APCI): m/z (positive mode): 382.8
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 5.08 min (HPLC purity: 98.8%).

### Example 58 : 4-(4-Nitro-thiophen-2-ylmethylene)-1-(3-trifluoromethyl-phenyl)-pyrazolidine-3,5-dione

The same procedure as employed in the preparation of Example 2 but starting from 4-nitrothiophene-2-carbaldehyde gave 15 mg of 4-(4-nitro-thiophen-2-ylmethylene)-1-(3-trifluoromethyl-phenyl)-pyrazolidine-3,5-dione as a red solid (52%).
¹H NMR (DMSO-d₆, 300 MHz) δ 11.68 (br s, 1H), 9.20 (s, 1H), 8.84 (m, 1H), 8.05-8.25 (m, 3H), 7.69 (m, 1H), 7.57 (m, 1H)
FI-MS (APCI): m/z (negative mode): 381.6
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 5.82 and 5.92 min (HPLC purity: 98%).

### Example 59 : 4-[3,5-Dioxo-1-(3-trifluoromethyl-phenyl)-pyrazolidin-4-ylidenemethyl]-thiophene-3-carboxylic acid

The same procedure as employed in the preparation of Example 2 but starting from 4-formyl-thiophene-3-carboxylic acid gave 8.5 mg of 4-[3,5-dioxo-1-(3-trifluoromethylphenyl)-pyrazolidin-4-ylidenemethyl]-thiophene-3-carboxylic acid as an orange solid (35%).
FI-MS (APCI): m/z (negative mode): 383.0
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 5.22 min (HPLC purity: 99.3%).

### Example 60 : {3-[3,5-Dioxo-1-(3-trifluoromethyl-phenyl)-pyrazolidin-4-ylidenemethyl]-thiophen-2-ylsulfanyl}-acetic acid

The same procedure as employed in the preparation of Example 2 but starting from 2-(2-oxo-propylsulfanyl)-thiophene-3-carbaldehyde gave 6.1 mg of {3-[3,5-dioxo-1-(3-trifluoromethyl-phenyl)-pyrazolidin-4-ylidenemethyl]-thiophen-2-ylsulfanyl} -acetic acid as an orange solid (26%).
FI-MS (APCI): m/z (negative mode): 428.8
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 5.32 and 5.52 min (HPLC purity: 95.6%).

### Example 61 : 2-[1-(4-Iodo-phenyl)-3,5-dioxo-pyrazolidin-4-ylidenemethyl]-thiophene-3-carboxylic acid

The same procedure as employed in the preparation of Example 1 but starting from 2-formyl-thiophene-3-carboxylic acid gave 27.8 mg of 2-[1-(4-iodo-phenyl)-3,5-dioxo-pyrazolidin-4-ylidenemethyl]-thiophene-3-carboxylic acid as a brown solid (74%).
¹H NMR (DMSO-d₆, 300 MHz) δ 13.59 (br s, 1H), 11.33 (m, 1H), 9.09 (m, 1H), 8.19 (m, 1H), 7.77 (m, 2H), 7.67-7.49 (m, 3H)
FI-MS (APCI): m/z (positive mode): 441.0
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 4.99 min (HPLC purity: 98.4%).

### Example 62: {3-[1-(4-Iodo-phenyl)-3,5-dioxo-pyrazolidin-4-ylidenemethyl]-thiophen-2-ylsulfanyl}-acetic acid

The same procedure as employed in the preparation of Example 1 but starting from 2-(2-oxo-propylsulfanyl)-thiophene-3-carbaldehyde gave 33.0 mg of {3-[1-(4-iodo-phenyl)-3,5-dioxo-pyrazolidin-4-ylidenemethyl]-thiophen-2-ylsulfanyl}-acetic acid as an orange solid (78%).
¹H NMR (DMSO-d₆, 300 MHz) δ 12.89 (br s, 1H), 11.32 (m, 1H), 8.70-8.50 (m, 1H), 8.04 (m, 1H), 7.78 (m, 3H), 7.58 (m, 2H), 3.87 (s, 2H)
FI-MS (APCI): m/z (positive mode): 486.8
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 5.19 and 5.40 min (HPLC purity: 98.2%).

### Example 63 : 4-(4-Hydroxymethyl-furan-3-ylmethylene)-1-(3-trifluoromethyl-phenyl)-pyrazolidine-3,5-dione

The same procedure as employed in the preparation of Example 2 but starting from 4-hydroxymethyl-furan-3-carbaldehyde gave 13 mg of 4-(4-hydroxymethyl-furan-3-ylmethylene)-1-(3-trifluoromethyl-phenyl)-pyrazolidine-3,5-dione as an orange solid (51%).
¹H NMR (DMSO-d₆, 300 MHz) δ 11.46 (br s, 1H), 9.45-9.20 (m, 1H), 8.24-8.04 (m, 2H), 7.94-7.78 (m, 2H), 7.69 (m, 1H), 7.55 (m, 1H), 5.28 (s, 1H), 4.56 (s, 2H)
FI-MS (APCI): m/z (positive mode): 350.8
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 4.60 and 4.97 min (HPLC purity: 98.1%).

### Example 64 : 4-Benzo[b]thiophen-2-ylmethylene-1-(3-trifluoromethyl-phenyl)-pyrazolidine-3,5-dione

The same procedure as employed in the preparation of Example 2 but starting from benzo[b]thiophene-2-carbaldehyde gave 16.7 mg of 4-benzo[b]thiophen-2-ylmethylene-1-(3-trifluoromethyl-phenyl)-pyrazolidine-3,5-dione as a brown solid (55%).
FI-MS (APCI): m/z (positive mode): 387.0 and m/z (negative mode): 389.0
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 6.45 and 6.48 min (HPLC purity: 92.8%).

### Example 65 : 5-[3,5-Dioxo-1-(3-trifluoromethyl-phenyl)-pyrazolidin-4-ylidenemethyl]-thiophene-3-carboxylic acid

The same procedure as employed in the preparation of Example 2 but starting from 5-formyl-thiophene-3-carboxylic acid gave 12.2 mg of 5-[3,5-dioxo-1-(3-trifluoromethylphenyl)-pyrazolidin-4-ylidenemethyl]-thiophene-3-carboxylic acid as a red solid (45%). ¹H NMR (DMSO-d₆, 300 MHz) δ 12.99 (br s, 1H), 8.79 (s, 1H), 8.57 (m, 1H), 7.85-8.20 (m, 3H), 7.69 (m, 1H), 7.55 (m, 1H)
FI-MS (APCI): m/z (positive mode): 380.8 and m/z (negative mode): 383.0
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 4.9 min (HPLC purity: 99.2%).

### Example 66 : 5-[1-(4-Iodo-phenyl)-3,5-dioxo-pyrazolidin-4-ylidenemethyl]-furan-2-carboxylic acid

The same procedure as employed in the preparation of Example 1 but starting from 5-formyl-furan-2-carboxylic acid gave 29.7 mg of 5-[1-(4-iodo-phenyl)-3,5-dioxo-pyrazolidin-4-ylidenemethyl]-furan-2-carboxylic acid as a dark purple powder (81%). FI-MS (Turboscan): m/z (negative mode): 422.8
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 4.63 and 4.79 min (HPLC purity: 86.1 %).

### Example 67 : 4-[4-(3-Dimethylamino-propylamino)-benzylidene]-1-(4-iodo-phenyl)-pyrazolidine-3,5-dione

The same procedure as employed in the preparation of Example 1 but starting from 4-formyl-N-(3-N,N-dimethylaminopropyl)aniline gave 29 mg of 4-[4-(3-dimethylamino-propylamino)-benzylidene]-1-(4-iodo-phenyl)-pyrazolidine-3,5-dione as an orange solid (69%).
FI-MS (Turboscan): m/z (positive mode): 491.0 and m/z (negative mode): 488.8
HPLC (C₈ Waters Symmetry Column, 4.6 x 50 mm², UV detection at 254 nm, 2 mL/min, 10 min gradient from 0.1 % TFA in H₂O to 0.07 % TFA in ACN), Rₜ: 3.85 min (HPLC purity: 95.9%).

### Example 68 : 1-(4-Iodo-phenyl)-4-(4-pyrrolidin-1-yl-benzylidene)-pyrazoliden-3,5-dione

The same procedure as employed in the preparation of Example 1 but starting from 4-pyrrolidin-1-yl-benzaldehyde gave 35 mg of 1-(4-iodo-phenyl)-4-(4-pyrrolidin-1-yl-benzylidene)-pyrazolidine-3,5-dione as a red powder (87%).
FI-MS (Turboscan): m/z (negative mode): 457.8
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 6.2 min (HPLC purity: 82.5%).

### Example 69 : 5-(1-Biphenyl-4-yl-3,5-dioxo-pyrazolidin-4-ylidenemethyl)-furan-2-carboxylic acid

The same procedure as employed in the preparation of Example 3 but starting from 5-formylfuran-2-carboxylic acid gave 24 mg of 5-(1-biphenyl-4-yl-3,5-dioxo-pyrazolidin-4-ylidenemethyl)-furan-2-carboxylic acid as a red powder (77%).
FI-MS (Turboscan): m/z (negative mode): 372.8
HPLC (C₈ Waters Symmetry Column, 4.6 x 50 mm², UV detection at 254 nm, 2 mL/min, 10 min gradient from 0.1% TFA in H₂O to 0.07 % TFA in ACN), Rₜ: 5.02 and 5.11 min (HPLC purity: 88.4%).

### Example 70 : 4-(4-Hydroxy-naphthalen-1-ylmethylene)-1-(4-iodo-phenyl)-pyrazolidine-3,5-dione

The same procedure as employed in the preparation of Example 1 but starting from 4-hydroxy-1-naphtaldehyde gave 25.1 mg of 4-(4-hydroxy-naphthalen-1-ylmethylene)-1-(4-iodo-phenyl)-pyrazolidine-3,5-dione as a red solid (66%).
¹H NMR (DMSO-d₆, 300 MHz) δ 11.84 (br s, 1H), 11.16 (m, 1H), 9.47 (m, 1H), 8.62 (s, 1H), 8.28 (d, 2H, J=8.3Hz), 7.76 (m, 3H), 7.60 (m, 3H), 7.06 (d, 1H, J=8.5Hz)
FI-MS (Turboscan): m/z (negative mode): 454.4
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 4.65 min (HPLC purity: 93.2%).

### Example 71 : 1-(4-Iodo-phenyl)-4-(1-oxy-pyridin-4-ylmethylene)-pyrazolidine-3,5-dione

The same procedure as employed in the preparation of Example 1 but starting from 4-pyridinecarboxaldehyde N-oxide gave 28.2 mg of 1-(4-iodo-phenyl)-4-(1-oxy-pyridin-4-ylmethylene)-pyrazolidine-3,5-dione as a red solid (81%).
FI-MS (Turboscan): m/z (negative mode): 405.6
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 3.09 min (HPLC purity: 83.8%).

### Example 72 : 2-(4-Furan-2-ylmethylene-3,5-dioxo-pyrazolidin-1-yl)-5-iodo-benzoic acid methyl ester

To a suspension of 2-(3,5-dioxo-pyrazolidin-1-yl)-5-iodo-benzoic acid methyl ester (Intermediate 4, 36 mg, 0.10 mmol) in EtOH (1.5 mL) were added furan-2-carbaldehyde (11.5 mg, 0.12 mmol) and one drop of pyridine under inert atmosphere. After stirring overnight at 70°C, the reaction mixture was cooled to rt and the orange solid precipitated out. Filtration on a fritté, washing with EtOH (2 x 1mL) and with Et₂O (3 x 1mL) and drying *in vacuo* at 40°C for 24h gave 16.5 mg of 2-(4-furan-2-ylmethylene-3,5-dioxo-pyrazolidin-1-yl)-5-iodo-benzoic acid methyl ester as an orange fine powder (48%).
¹H NMR (DMSO-d₆, 300 MHz) δ 11.32 (br s, 1H), 8.41 (d, 1H, J=3.8Hz), 8.26 (m, 1H), 8.02 (m, 2H), 7.64 (m, 1H), 7.36 (m, 1H), 6.92 (m, 1H), 3.72 (m, 3H)
FI-MS (Turboscan): m/z (negative mode): 436.8
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 3.84 and 3.93 min (HPLC purity: 84.5%).

### Example 73 : 1-(4-Iodo-phenyl)-4-[2-(4-methyl-piperazin-1-yl)-benzylidene]-pyrazolidine-3,5-dione

The same procedure as employed in the preparation of Example 1 but starting from 2-(4-methylpiperazino)benzaldehyde gave 17.1 mg of 1-(4-iodo-phenyl)-4-[2-(4-methyl-piperazin-1-yl)-benzylidene]-pyrazolidine-3,5-dione as a brown powder (45%).
¹H NMR (DMSO-d₆, 300 MHz) δ 12.0 (br s, 1H), 7.48 (d, 2H, J=8.8Hz), 7.27 (d, 2H, J=8.8Hz), 6.83 (t, 1H, J=7.1Hz), 6.71 (t, 2H, J=7.5Hz), 6.42 (t, 1H, J=7.1Hz), 3.73 (m, 1H), 3.10-3.40 (m, 2H), 3.00-2.75 (m, 4H), 2.25 (s, 3H), 2.10-2.26 (m, 1H), 1.85 (m, 1H)
FI-MS (Turboscan): m/z (negative mode): 486.6
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 3.06 min (HPLC purity: 92.6%).

### Example 74 : 4-(1H-Indol-5-ylmethylene)-1-(4-iodo-phenyl)-pyrazolidine-3,5-dione

The same procedure as employed in the preparation of Example 1 but starting from indole-5-carboxaldehyde gave 15.8 mg of 4-(1H-indol-5-ylmethylene)-1-(4-iodo-phenyl)-pyrazolidine-3,5-dione as a red powder (48%).
¹H NMR (DMSO-d₆, 300 MHz) δ 11.68 (s, 1H), 11.10 (s, 1H), 9.04 (bs, 0.7H iso A), 8.90 (bs, 0.3H iso B), 8.46 (dd, 1 H, J1=1.1 Hz, J2=8.9 Hz), 8.01 (bs, 1H), 7.75 (d, 2H, J=8.9 Hz), 7.45-7.70 (m, 4H), 6.64 (s, 1H)
FI-MS (Turboscan): m/z (negative mode): 427.6
HPLC (C₈ Waters Symmetry Column, 4.6 x 50 mm², UV detection at 254 nm, 2 mL/min, 8 min gradient from 0.1 % TFA in H₂O to 0.07 % TFA in ACN), Rₜ: 4.32 and 4.40 min (HPLC purity: 95.4%).

### Example 75 : 4-(4-Hydroxymethyl-furan-3-ylmethylene)-1-(4-iodo-phenyl)-pyrazolidine-3,5-dione

The same procedure as employed in the preparation of Example 1 but starting from 4-hydroxymethyl-furan-3-carbaldehyde gave 4.4 mg of 4-(4-hydroxymethyl-furan-3-ylmethylene)-1-(4-iodo-phenyl)-pyrazolidine-3,5-dione as a red powder (22%).
FI-MS (Turboscan): m/z (negative mode): 408.6
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 3.50 and 3.82 min (HPLC purity: 95.2%).

### Example 76 : 1-(4-Iodophenyl)-4-(1H-pyrrol-2-ylmethylene)-pyrazolidine-3,5-dione

The same procedure as employed in the preparation of Example 1 but starting from pyrrole-2-carboxaldehyde gave 19.5 mg of 1-(4-iodophenyl)-4-(1H-pyrrol-2-ylmethylene)-pyrazolidine-3,5-dione as an orange powder (64%).
¹H NMR (DMSO-d₆, 300 MHz) δ 13.32 (s, 1H), 11.40 (s, 1H), 7.81-7.68 (m, 4H), 7.55-7.48 (m, 2H), 7.32 (m, 1H), 6.53 (m, 1H)
FI-MS (Turboscan): m/z (negative mode): 377.4
HPLC (C₈ Waters Symmetry Column, 4.6 x 50 mm², UV detection at 254 nm, 2 mL/min, 8 min gradient from 0.1 % TFA in H₂O to 0.07 % TFA in ACN), Rₜ: 4.17 min (HPLC purity: 96.3%).

### Example 77 : 1-(4-Iodophenyl)-4-(1-methyl-1H-pyrrol-2-ylmethylene)-pyrazolidine-3,5-dione

The same procedure as employed in the preparation of Example 1 but starting from 1-methylpyrrole-2-carboxaldehyde gave 10.2 mg of 1-(4-iodophenyl)-4-(1-methyl-1H-pyrrol-2-ylmethylene)-pyrazolidine-3,5-dione as an orange powder (38%).
FI-MS (Turboscan): m/z (negative mode): 391.6
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 3.89 and 4.07 min (HPLC purity: 87.1 %).

### Example 78 : 1-(4-Iodo-phenyl)-4-[1-(2-nitro-benzyl)-1H-pyrrol-2-ylmethylene]-pyrazolidine-3,5-dione

The same procedure as employed in the preparation of Example 1 but starting from 1-(2-nitrobenzyl)pyrrole-2-carboxaldehyde gave 37.8 mg of 1-(4-iodo-phenyl)-4-[1-(2-nitrobenzyl)-1H-pyrrol-2-ylmethylene]-pyrazolidine-3,5-dione as a red powder (83%).
FI-MS (Turboscan): m/z (negative mode): 512.8
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 4.81 min (HPLC purity: 94.1%).

### Example 79 : 1-(4-Iodo-phenyl)-4-(4-pyridin-2-yl-benzylidene)-pyrazolidine-3,5-dione

The same procedure as employed in the preparation of Example 1 but starting from 4-(2-pyridyl)benzaldehyde gave 25.8 mg of 1-(4-iodo-phenyl)-4-(4-pyridin-2-yl-benzylidene)-pyrazolidine-3,5-dione as a brown powder (65%).
FI-MS (Turboscan): m/z (negative mode): 465.6
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 3.35 min (HPLC purity: 91.7%).

### Example 80 : 1-(4-Iodo-phenyl-4(1H-pyrazol-3-ylmethylene)-pyrazolidine-3,5-dione

The same procedure as employed in the preparation of Example 1 but starting from 1-H-imidazole-4-carbaldehyde gave 6 mg of 1-(4-iodo-phenyl-4(1H-pyrazol-3-ylmethylene)-pyrazolidine-3,5-dione as an orange powder (29%).
FI-MS (Turboscan): m/z (positive mode): 380.6 and m/z (negative mode): 378.4
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 2.49 and 3.14 min (HPLC purity: 96.4%).

### Example 81 : 1-(4-Iodo-phenyl-4(1H-pyrazol-3-ylmethylene)-pyrazolidine-3,5-dione

The same procedure as employed in the preparation of Example 1 but starting from pyrazol-3-carbaldehyde gave 15 mg of 1-(4-iodo-phenyl-4(1H-pyrazol-3-ylmethylene)-pyrazolidine-3,5-dione as a red powder (53%).
¹H NMR (DMSO-d₆, 300 MHz) δ 14.10 (br s, 0.6H, iso A), 13.90 (br s, 0.4H,iso B), 7.99 (m, 1H), 7.83 (s, 1H), 7.77 (m, 3H), 7.69-7.47 (m, 3H)
FI-MS (Turboscan): m/z (negative mode): 378.4
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 3.80 and 4.05 min (HPLC purity: 97.6%).

### Example 82 : 4-(2,3-Dihydro-benzofuran-5-ylmethylene)-1-(4-iodo-phenyl)-pyrazolidine-3,5-dione

The same procedure as employed in the preparation of Example 1 but starting from 2,3-dihydroI[b]furan-5-carbaldehyde gave 17.1 mg of 4-(2,3-dihydro-benzofuran-5-ylmethylene)-1-(4-iodo-phenyl)-pyrazolidine-3,5-dione as an orange powder (51%).
¹H NMR (DMSO-d₆, 300 MHz) δ 11.18 (br s, 1H), 8.80-8.60 (m, 1H), 8.47-8.35 (m, 1H), 7.80 (m, 1H), 7.76 (m, 2H), 7.56 (m, 2H), 6.97 (m, 1H), 4.71 (t, 2H, J=8.7Hz), 3.28 (t, 2H, J=8.7Hz)
FI-MS (Turboscan): m/z (negative mode): 430.6
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 4.61 min (HPLC purity: 93.9%).

### Example 83 : 1-(4-Iodo-phenyl)-4-quinolin-2-ylmethylene-pyrazolidine-3,5-dione

The same procedure as employed in the preparation of Example 1 but starting from quinoline-2-carbaldehyde gave 24.3 mg of 1-(4-iodo-phenyl)-4-quinolin-2-ylmethylene-pyrazolidine-3,5-dione as a brown powder (66%).
FI-MS (Turboscan): m/z (negative mode): 439.8
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 5.42 min (HPLC purity: 85.4%).

### Example 84 : 1-(4-Iodo-phenyl)-4-quinolin-4-ylmethylene-pyrazolidine-3,5-dione

The same procedure as employed in the preparation of Example 1 but starting from quinoline-4-carbaldehyde gave 14.4 mg of 1-(4-iodo-phenyl)-4-quinolin-4-ylmethylene-pyrazolidine-3,5-dione as a brown powder (44%).
FI-MS (Turboscan): m/z (negative mode): 439.4
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 2.94 min (HPLC purity: 84.1%).

### Example 85 : 4-[3-(2-Hydroxy-ethoxy)-benzylidene]-1-(4-iodo-phenyl)-pyrazolidine-3,5-dione

The same procedure as employed in the preparation of Example 1 but starting from 3-(2-hydroxyethoxy)benzaldehyde gave 8.8 mg of 4-[3-(2-hydroxy-ethoxy)-benzylidene]-1-(4-iodo-phenyl)-pyrazolidine-3,5-dione as a red solid (31%).
FI-MS (Turboscan): m/z (negative mode): 448.6
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 3.87 and 3.97 min (HPLC purity: 96.6%).

### Example 86 : 4-[4-(3-Dimethylamino-propoxy)-benzylidene]-1-(4-iodo-phenyl)-pyrazolidine-3,5-dione

The same procedure as employed in the preparation of Example 1 but starting from 4-(3'-N',N'-dimethylaminopropyloxy)benzaldehyde gave 34.2 mg of 4-[4-(3-dimethylamino-propoxy)benzylidene]-1-(4-iodo-phenyl)-pyrazolidine-3,5-dione as a brown solid (80%). FI-MS (Turboscan): m/z (positive mode): 492.0 and m/z (negative mode): 489.8
HPLC (C₈ Waters Symmetry Column, 4.6 x 50 mm² UV detection at 254 nm, 2 mL/min, 8 min gradient from 0.1% TFA in H₂O to 0.07 % TFA in ACN), Rₜ: 3.09 and 3.21 min (HPLC purity: 89.5%).

### Example 87 : 1-(4-Iodo-phenyl)-4-(5-naphthalen-2-yl-thiophen-2-ylmethylene)-pyrazolidine-3.5-dione

The same procedure as employed in the preparation of Example 1 but starting from 5-naphthalen-2-yl-thiophene-2-carbaldehyde gave 30 mg of 1-(4-iodo-phenyl)-4-(5-naphthalen-2-yl-thiophen-2-ylmethylene)-pyrazolidine-3,5-dione as an orange powder (67%).
¹H NMR (DMSO-d₆, 300 MHz) δ 11.19 (br s, 1H), 8.44-8.35 (m, 1H), 8.29-8.14 (m, 2H), 8.08 (m, 2H), 7.83-7.69 (m, 3H), 7.68-7.54 (m, 6H)
FI-MS (Turboscan): m/z (negative mode): 520.8
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 5.75 and 5.83 min (HPLC purity: 81 %).

### Example 88 : 4-(Diethylamino-hydroxy-benzylidene)-1-(4-iodo-phenyl)-pyrazolidine-3,5-dione

The same procedure as employed in the preparation of Example 1 but starting from 4-(diethylamino)salicyladehyde gave 20.7 mg of 4-(diethylamino-hydroxy-benzylidene)-1-(4-iodo-phenyl)-pyrazolidine-3,5-dione as a red solid (54%).
FI-MS (Turboscan): m/z (negative mode): 475.6
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 4.55 min (HPLC purity: 88.5%).

### Example 89 : 3-{5-[1-(4-Iodo-phenyl)-3,5-dioxo-pyrazolidin-4-ylidenemethyl]-furan-2-yl}-thiophene-2-carboxylic acid methyl ester

The same procedure as employed in the preparation of Example 1 but starting from 3-(5-formyl-furan-2-yl)-thiophene-2-carboxylic acid methyl ester gave 36.1 mg of 3-{5-[1-(4-iodo-phenyl)-3,5-dioxo-pyrazolidin-4-ylidenemethyl]-furan-2-yl}-thiophene-2-carboxylic acid methyl ester as a brown solid (79%).
FI-MS (Turboscan): m/z (negative mode): 518.8
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 5.28 min (HPLC purity: 94.2%).

### Example 90 : 1-(4-Iodo-phenyl)-4-[5-(2-nitro-4-trifluoromethyl-phenyl)-furan-2-yl-methylene]-pyrazolidine-3,5-dione

The same procedure as employed in the preparation of Example 1 but starting from 5-(2-nitro-4-trifluoromethyl-phenyl)-furan-2-carbaldehyde gave 11 mg of 1-(4-iodo-phenyl)-4-[5-(2-nitro-4-trifluoromethyl-phenyl)-furan-2-ylmethylene]-pyrazolidine-3,5-dione as a brown solid (28%).
¹H NMR (DMSO-d₆, 300 MHz) δ 11.3 (br s, 1H), 8.54 (m, 2H), 8.32 (d, 1H, J=8.3Hz), 8.23 (d, 1H, J=8.3Hz), 7.80 (dd, 2H, J1=2.3 Hz, J2=9.0 Hz), 7.65-7.48 (m, 4H)
FI-MS (Turboscan): m/z (negative mode): 567.6
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 5.63 min (HPLC purity: 96.7%).

### Example 91 : 4-(5-Chloro-thiophen-2-ylmethylene)-1-(4-iodo-phenyl)-pyrazolidine-3,5-dione

The same procedure as employed in the preparation of Example 1 but starting from 5-chlorothiophene-2-carbaldehyde gave 28.8 mg of 4-(5-chloro-thiophen-2-ylmethylene)-1-(4-iodo-phenyl)-pyrazolidine-3,5-dione as a red solid (79%).
FI-MS (Turboscan): m/z (negative mode): 428.6
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 4.73 and 5.03 min (HPLC purity: 88.2%).

### Example 92 : 1-(4-Iodo-phenyl)-4-thiazol-2-ylmethylene-pyrazolidine-3,5-dione

The same procedure as employed in the preparation of Example 1 but starting from thiazole-2-carbaldehyde gave 14.5 mg of 1-(4-iodo-phenyl)-4-thiazol-2-ylmethylene-pyrazolidine-3,5-dione as a black powder (49%).
FI-MS (APCI): m/z (positive mode): 397.8 and m/z (negative mode): 395.8
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 4.64 min (HPLC purity: 87.3%).

### Example 93 : 1-(4-Iodo-phenyl)-4-(1-methyl-1H-imidazol-2-ylmethylene)-pyrazolidine-3,5-dione

The same procedure as employed in the preparation of Example 1 but starting from 1-methyl-1H-imidazole-2-carbaldehyde gave 28.3 mg of 1-(4-iodo-phenyl)-4-(1-methyl-1H-imidazol-2-ylmethylene)-pyrazolidine-3,5-dione as a brown powder (84%).
¹H NMR (DMSO-d₆, 300 MHz) δ 7.77 (s, 1H), 7.74 (m, 4H), 7.63 (s, 1H), 7.57 (s, 1H), 4.02 (s, 3H)
FI-MS (APCI): m/z (positive mode): 395.0 and m/z (negative mode): 392.8
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 3.74 min (HPLC purity: 95.6%).

### Example 94 : 4-(2-Diethylamino-thiazol-5-ylmethylene)-1-(4-iodo-phenyl)-pyrazolidine-3,5-dione

The same procedure as employed in the preparation of Example 1 but starting from 2-(diethylamino)-1,3-thiazole-5-carbaldehyde gave 32.5 mg of 4-(2-diethylamino-thiazol-5-ylmethylene)-1-(4-iodo-phenyl)-pyrazolidine-3,5-dione as an orange powder (60%).
FI-MS (APCI): m/z (positive mode): 469.0 and m/z (negative mode): 466.8
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 4.33 min (HPLC purity: 90.8%).

### Example 95: 4-[2-(4-Benzyl:piperazin-1-yl)-thiazol-5-ylmethylene]-1-(4-iodo-phenyl)-pyrazolidine-3,5-dione

The same procedure as employed in the preparation of Example 1 but starting from 2-(4-benzylpiperazino)-1,3-thiazole-5-carbaldehyde gave 27.4 mg of 4-[2-(4-benzyl-piperazin-1-yl)-thiazol-5-ylmethylene]-1-(4-iodo-phenyl)-pyrazolidine-3,5-dione as an orange powder (57%).
FI-MS (APCI): m/z (positive mode): 572.0 and m/z (negative mode): 570.0
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 3.21 min (HPLC purity: 70.1%).

### Example 96 : 1-(4-Iodo-phenyl)-4-[2-(4-methoxy-phenoxy)-thiazol-5-ylmethylene]-pyrazolidine-3,5-dione

The same procedure as employed in the preparation of Example 1 but starting from 2-(4-methoxyphenoxy)-1,3-thiazaole-5-carbaldehyde gave 27.4 mg of 1-(4-iodo-phenyl)-4-[2-(4-methoxy-phenoxy)-thiazol-5-ylmethylene]-pyrazolidine-3,5-dione as an orange powder (62%).
¹H NMR (DMSO-d₆, 300 MHz) δ 11.23 (br s, 1H), 8.45 (m, 1H), 8.12 (m, 1H), 7.76 (m, 2H), 7.51 (m, 2H), 7.41 (m, 2H), 7.09 (m, 2H), 3.82 (s, 3H)
FI-MS (APCI): m/z (positive mode): 517.8 and m/z (negative mode): 520.0
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 4.86 min (HPLC purity: 98.2%).

### Example 97 : 4-(9-Ethyl-9H-carbazol-3-ylmethylene)-1-(4-iodo-phenyl)-pyrazolidine-3,5-dione

The same procedure as employed in the preparation of Example 1 but starting from 9-ethyl-H-carbazole-3-carbazole-3-carbaldehyde gave 24.8 mg of 4-(9-ethyl-9H-carbazol-3-ylmethylene)-1-(4-iodo-phenyl)-pyrazolidine-3,5-dione as an orange powder (59%).
¹H NMR (DMSO-d₆, 300 MHz) δ 11.15 (br s, 1H), 9.55-9.41 (m, 1H), 8.99-8.86 (m, 1H), 8.18 (m, 1H), 8.08 (m, 1H), 7.80 (m, 3H), 7.73 (m, 1H), 7.69-7.52 (m, 3H), 7.35 (m, 1H), 4.53 (q, 2H, J=7.1Hz), 1.36 (t, 3H, J=7.2Hz)
FI-MS (APCI): m/z (positive mode): 506.0 and m/z (negative mode): 508.0
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 5.52 min (HPLC purity: 91.3%).

### Example 98 : 4-Benzo[b]thiophen-3-ylmethylene-1-(4-iodo-phenyl)-pyrazolidine-3,5-dione

The same procedure as employed in the preparation of Example 1 but starting from benzo[b]thiophene-3-carbaldehyde gave 6.5 mg of 4-benzo[b]thiophen-3-yhnethylene-1-(4-iodo-phenyl)-pyrazolidine-3,5-dione as a brown powder (26%).
¹H NMR (DMSO-d₆, 300 MHz) δ11.45 (br s, 1H), 10.20 (m, 1H), 8.20 (m, 3H), 7.81 (m, 2H), 7.71-7.50 (m, 4H)
FI-MS (APCI): m/z (positive mode): 444.8 and m/z (negative mode): 447.0
HPLC (C₈ Waters Symmetry Column, 4.6 x 50 mm², UV detection at 254 nm, 2 mL/min, 8 min gradient from 0.1% TFA in H₂O to 0.07 % TFA in ACN), Rₜ: 5.10 and 5.30 min (HPLC purity: 95.5%).

### Example 99: Preparation of a pharmaceutical formulation

Pharmaceutical formulations using compounds of formula (I) may be prepared according to standard procedures known to a person skilled in the art.

The following formulation examples illustrate representative pharmaceutical compositions using compounds of formula (I).

### Formulation 1-Tablets

An alkylidene pyrazolidinedione of formula (I) is admixed as a dry powder with a dry gelatin binder in an approximate 1:2 weight ratio. A minor amount of magnesium stearate is added as a lubricant. The mixture is formed into 240-270 mg tablets (80-90 mg of active alkylidene pyrazolidinedione compound per tablet) in a tablet press.

### Formulation 2 - Capsules

An alkylidene pyrazolidinedione of formula (I) is admixed as a dry powder with a starch diluent in an approximate 1:1 weight ratio. The mixture is filled into 250 mg capsules (125 mg of active alkylidene pyrazolidinedione compound per capsule).

### Formulation 3 - Liquid

An alkylidene pyrazolidinedione of formula (1) (1250 mg), sucrose (1.75 g) and xanthan gum (4 mg) are blended, passed through a sieve having a 2 mm mesh size (a No. 10 mesh U.S. sieve) and then mixed with a previously prepared solution of microcrystalline cellulose and sodium carboxymethyl cellulose (11:89, 50 mg) in water. Sodium benzoate (10 mg), flavor, and color are diluted with water and added with stirring. Sufficient water is then added.

### Formulation 4 - Tablets

An alkylidene pyrazolidinedione of formula (1) is admixed as a dry powder with a dry gelatin binder in an approximate 1:2 weight ratio. A minor amount of magnesium stearate is added as a lubricant. The mixture is formed into 450-900 mg tablets (150-300 mg of active alkylidene pyrazolidinedione compound) in a tablet press.

### Formulation 5 - Injection

An alkylidene pyrazolidinedione of formula (I) is dissolved in a buffered sterile saline injectable aqueous medium to a concentration of approximately 5 mg/ml.

### Example 100: Biological assays

The compounds of formula (I) may be subjected to the following assays :
(1) The PTP Enzyme Assay
(2) The Insulin-Dependent Glucose Uptake Assay in C2C12 Cells
(3) The In vivo assay in db/db mice

### (1) The PTP Enzyme Assay (in vitro assay)

The PTP Enzyme Assay aims at determining the extent of inhibition of the examined PTP (e.g. PTP1B, TC-PTP, PTP-β, DEP-1, LAR, SHP-1, SHP-2, GLEPP-1, PTP-κ, PTP-µ, VHR, hVH5) in the presence of a test compound. The inhibition is illustrated by IC₅₀ values which denote the concentration necessary to achieve an inhibition of 50% of the given PTP.

### a) PTPs cloning

The cloning and expression of the catalytic domain of PTPs (e.g. PTP1B, TC-PTP, PTP-β, DEP-1, LAR, SHP-1, SHP-2, GLEPP-1, PTP-κ, PTP-µ, VHR, hVH5) may be performed as described in *J*. *Biol. Chem. 2000,* 275(13), pp 9792-9796.

### b) Materials and Methods

Assays were performed in a 96 well plate format, using the catalytic core of a human recombinant PTP, as an enzyme and 6,8-DiFluoro-4-MethylUmbelliferyl Phosphate (DiFMUP, Molecular Probes, D-6567) as a substrate at the Km value which was determined for each enzyme. Compounds to be tested were dissolved in 100% DMSO at a concentration of 2 mM. Subsequent dilutions of the test compounds (to yield a concentration of 100, 30, 10, 3, 1,0.3, 0.1, 0.03, 0.01, 0.001 µM) were performed in 100 % DMSO using a Tecan Stand Alone Workstation. 5 µl of diluted compound or vehicle (100% DMSO) was distributed to a black Costar 96 well plate. 25 µl of DiFMUP diluted in the assay buffer (20mM Tris HCl pH 7.5, 0.01% IGEPAL CA-630, 0.1mM ethylenediaminetetraacetic acid, 1mM DL-Dithiothreitol) were added, followed by 20µl of human recombinant PTP enzyme diluted in assay buffer in order to start the reaction. The reaction ran for 30 minutes at room temperature before reading the fluorescence intensity on a Perkin-Elmer Victor 2 spectrofluorimeter (excitation at 355nm, emission at 460 nm, for 0.1s). The percentage of inhibition relative to the fluorescence observed in the presence of solvent (5% DMSO) alone was determined. The IC50 values for inhibition were determined in triplicates on at least 3 separate occasions.

The tested compounds according to formula (I) display an inhibition (illustrated by IC₅₀ values) with regard to PTP1B, TC-PTP, SHP and GLEPP-1 of less than 10 µM, more preferred less than 1 µM. Compound 25 for instance displays an inhibition (IC₅₀) with regard to PTP1B of 40 nM, and of about 130 nM with regard to GLEPP-1. Compound 35 displays an inhibition (IC₅₀) with regard to PTP1B of 580 nM. Compound 45 displays an inhibition (IC₅₀) with regard to GLEPP-1 of 47 nM.

### (2) The Insulin-Dependent Glucose Uptake Assay of C2C12 Cells (Cell Assay)

The following cell assay aims at determining the effect of the test compounds on the insulin-induced glucose uptake by C2C12 cells, using Cytostar-T Scintillating Microplates from Amersham^{™}. The use of such Cytostar-T scintillating microplates for the determination of the glucose uptake was disclosed in Proximity News Issue C4 of May 1996 for 3T3-L1 adipocytes. For the present invention a modified assay was employed to monitor the glucose uptake by a different cell type, i.e. by C2C12 cells.

The principle of said commercially available Cytostar-T scintillating microplates is the following : The Cytostar-T scintillating microplates comprise a transparent scintillating base plate wherein a monolayer of adherent cells is grown. In order words, the scintillation agents which are to be activated by the radiolabel (i.e. 2-deoxy-D-[U-¹⁴C]glucose in the instant case) necessary for the measurement are integrated into the base plate. The decay of the free radiolabel in the growth medium is too distant to produce a signal that may be measured. However, upon penetration of the radiolabel into the cells, their decay will give rise to a signal owing to the proximity of said radiolabel to the scintillating base plate.

Thus, the quantification of the scintillation proximity signal allows the evaluation of the glucose up-take.

Day 1 : The assay is performed with commercially available Cytostar-T scintillating microplates whereby C2C12 cells are grown thereon at 8*10e4 /ml in a growth medium (DMEM High Glc, Gln, Penstrep 10% FCS). 100µl/wells in a 96 well-plate are used.
Day 3 : 100 µl of a differentiation medium (DMEM- High Glc 2% HS GIn Penstrep) is added to the wells.
Day 4 : The medium is changed.
Day 7 : The medium is removed and incubated in SSM buffer for 24h (DMEM, 0.5% BSA, Gln, Pen strep).
Day 8: The cells are then ready for the determination of the glucose uptake assay :

The medium is aspirated. 50µl of experimental medium (1/4DMEM-low Glc; 2mM Gln, ¾ PBS) 2h 37°C is added. By this step the cells are glucose-starved.

50µl of the test compound in experimental medium (0.2% DMSO) is added at different concentrations (0.05 µM; 0.1 µM; 0.3. µM; 1 µM; 3 µM; 5 µM; 10 µM and 30 µM). The plate is kept at 37°C;
For the positive control, the reference compound Cytochalasin B (which is a known agent that inhibits the glucose transport and which is available from SIGMA C-6762) in experimental medium (0.2% DMSO) is added.

For the negative control, 50µl of experimental medium (0.2% DMSO) is added,

After about 20 minutes, Insulin (bovine) is added to the control wells to 200nM to trigger the signalling pathway (37°C);

After 10 minutes 5 µl of the radiolabel 2-deoxy-D-[U-¹⁴C]glucose (having a concentration of 0.1 µCi final per well) (in 5µl experimental medium; 37°C) is added. The test compound inhibits the action of PTP 1B so that the uptake of glucose (i.e. of the radiolabel 2-deoxy-D-[U-¹⁴C]glucose) by the C2C12 cells is stimulated. The radiolabel, now incorporated into the cells is close or in contact with the scintillating base plate, containing the scintillation agents incorporated into the well. The quantification of the scintillation proximity signal allows the evaluation of the glucose up-take. Such scintillation may be measured by a suitable counter; in the instant case, the plate is read on a Wallac Trilux scintillation counter (Window setting 5-500).

The tested compounds according to formula (I) display an average stimulation (%) of glucose uptake in muscle cells of between 10-30% at a concentration of 1 µM.

## Claims

1. Use of an alkylidene pyrazolidinedione derivative according to formula (I) 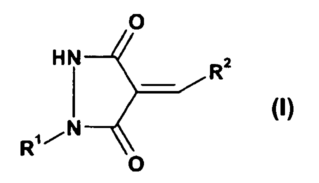 as well as its tautomers, geometrical isomers, its optically active forms as enantiomers, diastereomers and its racemate forms, as well as pharmaceutically acceptable salts thereof, wherein
R¹ is an aryl and R² represent an aryl or heteroaryl, for the preparation of a pharmaceutical composition for the treatment and/or prevention of metabolic disorders mediated by insulin resistance or hyperglycemia, namely diabetes type I and/or II, obesity and polycystic ovary syndrome (PCOS).

2. Use according to claim 1 for the treatment and/or prevention of diabetes type II.

3. Use according to claim 1 or 2, wherein R² is a furanyl, thienyl, pyrrolyl, indolyl, quinolyl or carbazolyl which is optionally substituted by a C₁-C₆ alkyl, C₂-C₆ alkenyl, C₂-C₆ alkynyl, aryl or heteroaryl, 3-8 membered cycloalkyl or heterocycloalkyl, amino, sulfonyl, sulfanyl, sulfinyl, C₁-C₆-alkyl sulfonyl, C₁-C₆-alkyl sulfinyl, C₁-C₆-alkyl sulfanyl, alkoxy, halogen, including an iodo, cyano, carboxy, alkoxycarbonyl or nitro group.

4. Use according to claim 3, wherein R² is a furanyl, thienyl, pyrrolyl, indolyl, quinolyl or carbazolyl, which is optionally substituted by a halogen, carboxy, alkoxycarbonyl or nitro group.

5. Use according to any of the preceding claims, wherein R² is an unsubstituted or substituted furanyl.

6. Use according to any of the preceding claims, wherein R¹ is a substituted phenyl.

7. Use according to claim 6, wherein R¹ is a iodophenyl, trifluoromethylphenyl, chloro-methylphenyl, iodobenzoic acid methyl ester, or a biphenyl group.

8. An alkylidene pyrazolidinedione derivative selected from the group consisting of:
4-(4-Iodo-benzylidene)-1-(4-iodo-phenyl)-pyrazolidine-3,5-dione
4-(Dimethyl-furan-2-ylmethylene)-1-(3-trifluoromethyl-phenyl)-pyrazolidine-3,5-dione
1-Biphenyl-4-yl-4-furan-2-ylmethylene-pyrazolidine-3,5-dione
Acetic acid 5-[1-(4-iodo-phenyl)-3,5-dioxo-pyrazolidin-4-ylidenemethyl]-furan-2-ylmethyl ester
4-(4-Bromo-furan-2-ylmethylene)-1-(4-iodo-phenyl)-pyrazolidine-3,5-dione
1-(4-Iodo-phenyl)-4-(5-methyl-furan-2-yl methyl ene)-pyrazolidine-3, 5-dione
4-(5-Bromo-furan-2-ylmethylene)-1-(4-iodo-phenyl)-pyrazolidine-3,5-dione
1-(4-Iodo-phenyl)-4-[5-(2-methoxy-phenyl)-thiophen-2-ylmethylene]-pyrazolidine-3,5-dione
1-(4-Iodo-phenyl)-4-thiophen-3-ylmethylene-pyrazolidine-3,5-dione
1-(4-Iodo-phenyl)-4-(5-phenyl-thiophen-2-ylmethylene)-pyrazolidine-3,5-dione
4-Furan-2-ylmethylene-1-(3-trifluoromethyl-phenyl)-pyrazolidine-3,5-dione
4-(5-Methyl-furan-2-ylmethylene)-1-(3-trifluoromethyl-phenyl)-pyrazolidine-3,5-dione
4-Thiophen-2-ylmethylene-1-(3-trifluoromethyl-phenyl)-pyrazolidine-3,5-dione
4-(4-Bromo-furan-2-ylmethylene)-1-(3-trifluoromethyl-phenyl)-pyrazolidine-3,5-dione
Acetic acid 5-[3,5-dioxo-1-(3-trifluoromethyl-phenyl)-pyrazolidin-4-ylidenemethyl]-furan-2-ylmethyl ester
1-(3-Chloro-4-methyl-phenyl)-4-furan-2-ylmethylene-pyrazolidine-3, 5-dione
4-(7-Bromo-8-hydroxy-quinolin-5-ylmethylene)-1-(4-iodo-phenyl)-pyrazolidine-3,5-dione
1-(4-Iodo-phenyl)-4-[5-(4-nitro-phenyl)-furan-2-ylmethylene]-pyrazolidine-3,5-dione
1-(4-Iodo-phenyl)-4-[5-(2-nitro-phenyl)-furan-2-ylmethylene]-pyrazolidine-3,5-dione
4-(5-Hydroxymethyl-furan-2-ylmethylene)-1-(4-iodo-phenyl)-pyrazolidine-3,5-dione
4-((E)-3-Furan-2-yl-allylidene)-1-(4-iodo-phenyl)-pyrazolidine-3,5-dione
4-Furan-3-ylmethylene-1-(4-iodo-phenyl)-pyrazolidine-3,5-dione
1-(4-Iodo-phenyl)-4-(5-nitro-thiophen-2-ylmethylene)-pyrazolidine-3,5-dione
4-(5-Hydroxymethyl-furan-2-ylmethylene)-1-(3-trifluoromethyl-phenyl)-pyrazolidine-3,5-dione
4-Furan-3-ylmethylene-1-(3-trifluoromethyl-phenyl)-pyrazolidine-3,5-dione
4-(5-Nitro-thiophen-2-ylmethylene)-1-(3-trifluoromethyl-phenyl)-pyrazolidine-3,5-dione
1-(4-Iodo-phenyl)-4-(5-nitro-furan-2-ylmethylene)-pyrazolidine-3,5-dione
1-(4-Iodo-phenyl)-4-[5-(3-nitro-phenyl)-furan-2-ylmethylene]-pyrazolidine-3,5-dione
4-(4,5-Dimethyl-furan-2-ylmethylene)-1-(4-iodo-phenyl)-pyrazolidine-3,5-dione
5-[1-(4-Iodo-phenyl)-3,5-dioxo-pyrazolidin-4-ylidenemethyl]-furan-2-sulfonic acid
4-[1-(4-Iodo-phenyl)-3,5-dioxo-pyrazolidin-4-ylidenemethyl]-benzonitrile
1-(4-Iodo-phenyl)-4-(3-methyl-thiophen-2-ylmethylene)-pyrazolidine-3,5-dione
4-(4-Bromo-thiophen-2-ylmethylene)-1-(4-iodo-phenyl)-pyrazolidine-3,5-dione
1-(4-Iodo-phenyl)-4-(5-methyl-thiophen-2-ylmethylene)-pyrazolidine-3,5-dione
4-Benzo[b]thiophen-2-ylmethylene-1-(4-iodo-phenyl)-pyrazolidine-3,5-dione
1-(4-Iodo-phenyl)-4-(4-nitro-thiophen-2-ylmethylene)-pyrazolidine-3,5-dione
5-[1-(4-Iodo-phenyl)-3,5-dioxo-pyrazolidin-4-ylidenemethyl]-thiophene-3-carboxylic acid
4-[1-(4-Iodo-phenyl)-3,5-dioxo-pyrazolidin-4-ylidenemethyl]-thiophene-3-carboxylic acid
2-[3,5-Dioxo-1-(3-trifluoromethyl-phenyl)-pyrazolidin-4-ylidenemethyl]-thiophene-3-carboxylic acid
4-(4-Nitro-thiophen-2-ylmethylene)-1-(3-trifluoromethyl-phenyl)-pyrazolidine-3,5-dione
4-[3,5-Dioxo-1-(3-trifluoromethyl-phenyl)-pyrazolidin-4-ylidenemethyl]-thiophene-3-carboxylic acid
{3-[3,5-Dioxo-1-(3-trifluoromethyl-phenyl)-pyrazolidin-4-ylidenemethyl]-thiophen-2-ylsulfanyl}-acetic acid
2-[1-(4-Iodo-phenyl)-3,5-dioxo-pyrazolidin-4-ylidenemethyl]-thiophene-3-carboxylic acid
{3-[1-(4-Iodo-phenyl)-3,5-dioxo-pyrazolidin-4-ylidenemethyl]-thiophen-2-ylsulfanyl}-acetic acid
4-(4-Hydroxymethyl-furan-3-ylmethylene)-1-(3-trifluoromethyl-phenyl)-pyrazolidine-3,5-dione
4-benzo[b]thiophen-2-ylmethylene-1-(3-trifluoromethyl-phenyl)-pyrazolidine-3,5-dione
5-[3,5-Dioxo-1-(3-trifluoromethyl-phenyl)-pyrazolidin-4-ylidenemethyl]-thiophene-3-carboxylic acid
5-[1-(4-Iodo-phenyl)-3,5-dioxo-pyrazolidin-4-ylidenemethyl]-furan-2-carboxylic acid
5-(1-Biphenyl-4-yl-3,5-dioxo-pyrazolidin-4-ylidenemethyl)-furan-2-carboxylic acid
4-(4-Hydroxy-naphthalen-1-ylmethylene)-1-(4-iodo-phenyl)-pyrazolidine-3,5-dione
1-(4-Iodo-phenyl)-4-(1-oxy-pyridin-4-ylmethylene)-pyrazolidine-3,5-dione
2-(4-Furan-2-ylmethylene-3,5-dioxo-pyrazolidin-1-yl)-5-iodo-benzoic acid methyl ester
4-(1H-Indol-5-ylmethylene)-1-(4-iodo-phenyl)-pyrazolidine-3,5-dione
4-(4-Hydroxymethyl-furan-3-ylmethylene)-1-(4-iodo-phenyl)-pyrazolidine-3,5-dione
1-(4-Iodophenyl)-4-(1H-pyrrol-2-ylmethylene)-pyrazolidine-3,5-dione
1-(4-Iodophenyl)-4-(1-methyl-1 H-pyrrol-2-ylmethylene)-pyrazolidine-3,5-dione
1-(4-Iodo-phenyl)-4-[1-(2-nitro-benzyl)-1H-pyrrol-2-ylmethylene]-pyrazolidine-3,5-dione
1-(4-Iodo-phenyl-4(1H-pyrazol-3-ylmethylene)-pyrazolidine-3,5-dione
1-(4-Iodo-phenyl-4(1H-pyrazol-3-ylmethylene)-pyrazolidine-3,5-dione
4-(2,3-Dihydro-benzofuran-5-ylmethylene)-1-(4-iodo-phenyl)-pyrazolidine-3, 5-dione
1-(4-Iodo-phenyl)-4-quinolin-2-ylmethylene-pyrazolidine-3,5-dione
1-(4-Iodo-phenyl)-4-quinolin-4-ylmethylene-pyrazolidine-3,5-dione
4-[3-(2-Hydroxy-ethoxy)-benzylidene]-1-(4-iodo-phenyl)-pyrazolidine-3,5-dione
1-(4-Iodo-phenyl)-4-(5-naphthalen-2-yl-thiophen-2-ylmethylene)-pyrazolidine-3,5-dione
3-{5-[1-(4-Iodo-phenyl)-3,5-dioxo-pyrazolidin-4-ylidenemethyl]-furan-2-yl}-thiophene-2-carboxylic acid methyl ester
1-(4-Iodo-phenyl)-4-[5-(2-nitro-4-trifluoromethyl-phenyl)-furan-2-ylmethylene]-pyrazolidine-3,5-dione
4-(5-Chloro-thiophen-2-ylmethylene)-1-(4-iodo-phenyl)-pyrazolidine-3,5-dione
1-(4-Iodo-phenyl)-4-thiazol-2-ylmethylene-pyrazolidine-3,5-dione
1-(4-Iodo-phenyl)-4-(1-methyl-1H-imidazol-2-ylmethylene)-pyrazolidine-3,5-dione
4-(2-Diethylamino-thiazol-5-ylmethylene)-1-(4-iodo-phenyl)-pyrazolidine-3,5-dione
4-[2-(4-Benzyl-piperazin-1-yl)-thiazol-5-ylmethylene]-1-(4-iodo-phenyl)-pyrazolidine-3,5-dione
1-(4-Iodo-phenyl)-4-[2-(4-methoxy-phenoxy)-thiazol-5-ylmethylene]-pyrazolidine-3,5-dione
4-(9-Ethyl-9H-carbazol-3-ylmethylene)-1-(4-iodo-phenyl)-pyrazolidine-3,5-dione
4-Benzo[b]thiophen-3-ylmethylene-1-(4-iodo-phenyl)-pyrazolidine-3,5-dione

9. An alkylidene pyrazolidinedione derivative selected from the group consisting of:
4-(2-Hydroxy-benzylidene)-1-(4-iodo-phenyl)-pyrazolidine-3,5-dione
4-[1-(4-Iodo-phenyl)-3,5-dioxo-pyrazolidin-4-ylidenemethyl]-benzoic acid
{4-[1-(4-Iodo-phenyl)-3,5-dioxo-pyrazolidin-4-ylidenemethyl]-phenoxy}-acetic acid
(E)-3-{4-[1-(4-Iodo-phenyl)-3,5-dioxo-pyrazolidin-4-ylidenemethyl]-phenyl} -acrylic acid
2-Hydroxy-5-[1-(4-iodo-phenyl)-3,5-dioxo-pyrazolidin-4-ylidenemethyl]-benzoic acid
3-[1-(4-Iodo-phenyl)-3,5-dioxo-pyrazolidin-4-ylidenemethyl]-benzoic acid
(E)-3-{4-[3,5-Dioxo-1-(3-trifluoromethyl-phenyl)-pyrazolidin-4-ylidenemethyl]-phenyl}-acrylic acid
{2-[1-(4-Iodo-phenyl)-3,5-dioxo-pyrazolidin-4-ylidenemethyl]-phenoxy}-acetic acid
{4-[3,5-Dioxo-1-(3-trifluoromethyl-phenyl)-pyrazolidin-4-ylidenemethyl]-phenoxy} - acetic acid
5-[3,5-Dioxo-1-(3-trifluoromethyl-phenyl)-pyrazolidin-4-ylidenemethyl]-2-hydroxybenzoic acid
{2-[3,5-Dioxo-1-(3-trifluoromethyl-phenyl)-pyrazolidin-4-ylidenemethyl]-phenoxy}-acetic acid
4-[4-(3-Dimethylamino-propylamino)-benzylidene]-1-(4-iodo-phenyl)-pyrazolidine-3,5-dione
1-(4-Iodo-phenyl)-4-(4-pyrrolidin- I -yl-benzylidene)-pyrazolidine-3,5-dione
1-(4-Iodo-phenyl)-4-[2-(4-methyl-piperazin-1-yl)-benzylidene]-pyrazolidine-3,5-dione
1-(4-Iodo-phenyl)-4-(4-pyridin-2-yl-benzyl idene)-pyrazol idine-3, 5-dione
4-[4-(3-Dimethylamino-propoxy)-benzylidene]-1-(4-iodo-phenyl)-pyrazolidine-3,5-dione
4-(Diethylamino-hydroxy-benzylidene)-1-(4-iodo-phenyl)-pyrazolidine-3,5-dione.

10. A alkylidene pyrazolidinedione derivative according to any of claims 8 to 9 for use as a medicament.

11. A pharmaceutical composition containing at least one alkylidene pyrazolidinedione derivative according to any of claims 8 to 9 and a pharmaceutically acceptable carrier, diluent or excipient thereof.

12. A method of preparing compounds of formula (III) 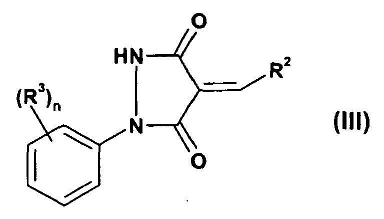 comprising the step of reacting precursor (IV) with the aldehyde (V)

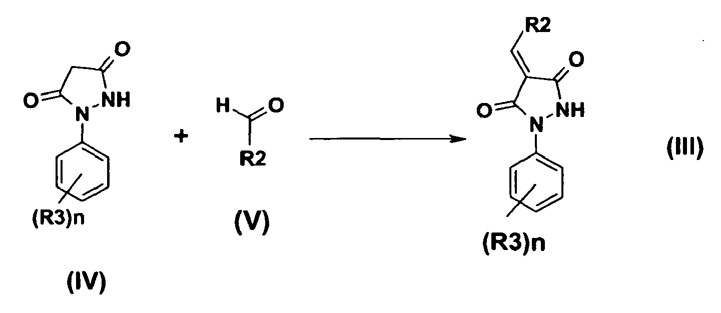

## Patentansprüche

1. Verwendung eines Alkylidenpyrazolidindion-Derivats entsprechend Formel (I) 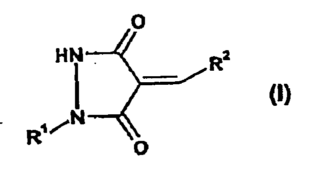 als auch seiner Tautomere, geometrischen Isomere, seiner aktiven Formen wie Enantiomeren, Diastereomeren und seiner Recematformen, als auch pharmazeutisch verträglicher Salze davon, worin
R¹ ein Aryl ist und R² ein Aryl oder Heteroaryl bedeutet, zur Herstellung einer pharmazeutischen Zusammensetzung zur Behandlung und oder Verhinderung von Stoffwechselstörungen, die vermittelt werden durch Insulinresistenz oder Hyperglykämie, nämlich Diabetes Typ I und/oder Typ II, Obesität und polyzystisches Ovarsyndrom (PCOS).

2. Verwendung nach Anspruch 1 zur Behandlung und/oder Verhinderung von Diabets Typ II.

3. Verwendung nach Anspruch 1 oder 2, worin R² ein Furanyl, Thienyl, Pyrrolyl, Indolyl, Chinolyl oder Carbazolyl ist, das optional substituiert ist durch ein C₁-C₆₋Alkyl, C₂-C₆-Alkenyl, C₂-C₆-Alkinyl, Aryl oder Heteroaryl, 3-8-gliedriges Cycloalkyl oder Heterocyclocycloalkyl, Amino, Sulfonyl, Sulfanyl, Sulfinyl, C₁-C₆-Alkylsulfonyl, C₁-C₆-Alkylsulfinyl, C₁-C₆-Alkylsulfanyl, Alkoxy, Halogen, einschließlich einer lod-, Cyano-, Carboxy-, Alkoxycarbonyl- oder Nitro-Gruppe.

4. Verwendung nach Anspruch 3, worin R² Furanyl, Thienyl, Pyrrolyl, Indolyl, Chinolyl oder Carbazolyl ist, das optional substituiert ist durch eine Halogen-, Carboxy-, Alkoxycarbonyl- oder Nitro-Gruppe.

5. Verwendung nach einem der vorhergehenden Ansprüche, worin R² ein unsubstituiertes oder substituiertes Furanyl ist.

6. Verwendung nach einem der vorhergehenden Ansprüche, worin R¹ ein substituiertes Phenyl ist.

7. Verwendung nach Anspruch 6, worin R¹ eine Iodphenyl-, Trifluormethylphenyl-, Chlormethylphenyl-, lodbenzoesäuremethylester- oder eine Biphenylgruppe ist.

8. Alkylidenpyrazolidindion-Derivat, ausgewählt aus der Gruppe, bestehend aus:
4-(4-lod-benzyliden)-1-(4-lod-phenyl)-pyrazolidin-3,5-dion,
4-(Dimethyl-furan-2-ylmethylen)-1-(3-trifluormethyl-phenyl)-pyrazolidin-3,5-dion,
1-Biphenyl-4-yl-4-furan-2-ylmethylen-pyrazolidin-3,5-dion,
Essigsäure-5-[1-(4-iod-phenyl)-3,5-dioxo-pyrazolidin-4-ylidenmethyl]-furan-2-ylmethyl-ester,
4-(4-Bromo-furan-2-ylmethylen)-1-(4-iod-phenyl)-pyrazolidin-3,5-dion,
1-(4-Iodo-phenyl)-4-(5-methyl-furan-2-ylmethylen)-pyrazolidin-3,5-dion,
4-(5-Brom-furan-2-ylmethylen)-1-(4-iod-phenyl)-pyrazolidin-3,5-dion,
1-(4-lod-phenyl)-4-[5-(2-methoxy-phenyl)-thiophen-2-ylmethylen]pyrazolidin-3,5-dion,
1-(4-lod-phenyl)-4-thiophen-3-ylmethylen-pyrazolidin-3,5-dion,
1-(4-lod-phenyl)-4-(5-phenyl-thiophen-2-ylmethylen)-pyrazolidin-3,5-dion,
4-Furan-2-ylmethylen-1-(3-trifluoromethyl-phenyl)-pyrazolidin-3,5-dion,
4-(5-Methyl-furan-2-ylmethylen)-1-(3-trifluoromethyl-phenyl)-pyrazolidin-3,5-dion,
4-Thiophen-2-ylmethylen-1-(3-trifluormethyl-phenyl)-pyrazolidin-3,5-dion,
4-(4-Brom-furan-2-ylmethylen)-1-(3-trifluoromethyl-phenyl)-pyrazolidin-3,5-dion,
Essigsäure-5-[3,5-dioxo-1-(3-trifluoromethyl-phenyl)-pyrazolidin-4-ylidenmethyl]furan-2-ylmethyl-ester,
1-(3-Chlor-4-methyl-phenyl)-4-furan-2-ylmethylen-pyrazolidin-3,5-dion,
4-(7-Brom-8-hydroxy-chinolin-5-ylmethylen)-1-(4-iod-phenyl)-pyrazolidin-3,5-dion,
1-(4-lod-phenyl)-4-[5-(4-nitro-phenyl)-furan-2-ylmethylen]-pyrazolidin-3,5-dion,
1-(4-lod-phenyl)-4-[5-(2-nitro-phenyl)-furan-2-ylmethylen]-pyrazolidin-3,5-dion,
4-(5-Hydroxymethyl-furan-2-ylmethylen)-1-(4-iod-phenyl)-pyrazolidin-3,5-dion,
4-((E)-3-Furan-2-yl-allyliden)-1-(4-iod-phenyl)-pyrazolidin-3,5-dion,
4-Furan-3-ylmethylen-1-(4-iod-phenyl)-pyrazolidin-3,5-dion,
1-(4-Iod-phenyl)-4-(5-nitro-thiophen-2-ylmethylen)-pyrazolidin-3,5-dion,
4-(5-Hydroxymethyl-furan-2-ylmethylen)-1-(3-trifluormethyl-phenyl)-pyrazolidin-3,5-dion,
4-Furan-3-ylmethylen-1-(3-trifluoromethyl-phenyl)-pyrazolidin-3,5-dion,
4-(5-Nitro-thiophen-2-ylmethylen)-1-(3-trifluoromethyl-phenyl)-pyrazolidin-3,5-dion,
1-(4-lod-phenyl)-4-(5-nitro-furan-2-ylmethylen)-pyrazolidin-3,5-dion,
1-(4-lod-phenyl)-4-[5-(3-nitro-phenyl)-furan-2-ylmethylen]-pyrazolidin-3,5-dion,
4-(4,5-Dimethyl-furan-2-ylmethylen)-1-(4-iod-phenyl)-pyrazolidin-3,5-dion,
5-[1-(4-lod-phenyl)-3,5-dioxo-pyrazolidin-4-ylidenmethyl]-furan-2-sulfonsäure,
4-[1-(4-lod-phenyl)-3,5-dioxo-pyrazolidin-4-ylidenmethyl]-benzonitril,
1-(4-Iod-phenyl)-4-(3-methyl-thiophen-2-ylmethylen)-pyrazolidin-3,5-dion,
4-(4-Brom-thiophen-2-ylmethylen)-1-(4-iod-phenyl)-pyrazolidin-3,5-dion,
1-(4-lod-phenyl)-4-(5-methyl-thiophen-2-ylmethylen)-pyrazolidin-3,5-dion,
4-Benzo[b]thiophen-2-ylmethylen-1-(4-iodphenyl)-pyrazolidin-3,5-dion,
1-(4-lod-phenyl)-4-(4-nitro-thiophen-2-ylmethylen)-pyrazolidin-3,5-dion,
5-[1-(4-lod-phenyl)-3,5-dioxo-pyrazolidin-4-ylidenmethyl]-thiophen-3-carbonsäure,
4-[1-(4-lod-phenyl)-3,5-dioxo-pyrazolidin-4-ylidenmethyl]-thiophen-3-carbonsäure,
2-[3,5-Dioxo-1-(3-trifluoromethyl-phenyl)-pyrazolidin-4-ylidenmethyl]-thiophen-3-cabonsäure,
4-(4-Nitro-thiophen-2-ylmethylen)-1-(3-trifluoromethyl-phenyl)-pyrazolidin-3,5-dion,
4-[3,5-Dioxo-1-(3-trifluoromethyl-phenyl)-pyrazolidin-4-ylidenmethyl]-thiophen-3-carbonsäure,
{3-[3,5-Dioxo-1-(3-trifluoromethyl-phenyl)-pyrazolidin-4-ylidenmethyl]-thiophen-2-ylsulfanyl}-essigsäure,
2-[1-(4-lod-phenyl)-3,5-dioxo-pyrazolidin-4-ylidenmethyl]-thiophen-3-carbonsäure,
{3-[1-(4-Iod-phenyl)-3,5-dioxo-pyrazolidin-4-ylidenmethyl]-thiophen-2-ylsulfanyl}-essigsäure,
4-(4-Hydroxymethyl-furan-3-ylmethylen)-1-(3-trifluoromethyl-phenyl)-pyrazolidin-3,5-dion,
4-benzo[b]thiophen-2-ylmethylen-1-(3-trifluormethyl-phenyl)-pyrazolidin-3,5-dion,
5-[3,5-Dioxo-1-(3-trifluoromethyl-phenyl)-pyrazolidin-4-ylidenmethyl]-thiophen-3-carbonsäure,
5-[1-(4-lod-phenyl)-3,5-dioxo-pyrazolidin-4-ylidenmethyl]-furan-2-carbonsäure,
5-(1-Biphenyl-4-yl-3,5-dioxo-pyrazolidin-4-ylidenmethyl)-furan-2-carbonsäure,
4-(4-Hydroxy-naphthalin-1-ylmethylen)-1-(4-iod-phenyl)-pyrazolidin-3,5-dion,
1-(4-lod-phenyl)-4-(1-oxy-pyridin-4-ylmethylen)-pyrazolidin-3,5-dion,
2-(4-Furan-2-ylmethylen-3,5-dioxo-pyrazolidin-1-yl)-5-iod-benzoesäuremethylester,
4-(1H-Indol-5-ylmethylen)-1-(4-iod-phenyl)-pyrazolidin-3,5-dion,
4-(4-Hydroxymethyl-furan-3-ylmethylen)-1-(4-iod-phenyl)-pyrazolidin-3,5-dion,
1-(4-lod-phenyl)-4-(1H-pyrrol-2-ylmethylen)-pyrazolidin-3,5-dion,
1-(4-lod-phenyl)-4-(1-methyl-1H-pyrrol-2-ylmethylen)-pyrazolidin-3,5-dion,
1-(4-lod-phenyl)-4-[1-(2-nitro-benzyl)-1H-pyrrol-2-ylmethylen]-pyrazolidin-3,5-dion,
1-(4-Iod-phenyl)-4-(1H-pyrazol-3-ylmethylen)-pyrazolidin-3,5-dion,
1-(4-Iod-phenyl)-4-(1H-pyrazol-3-ylmethylen)-pyrazolidin-3,5-dion,
4-(2,3-Dihydro-benzofuran-5-ylmethylen)-1-(4-iod-phenyl)-pyrazolidin-3,5-dion,
1-(4-Iod-phenyl)-4-chinolin-2-ylmethylen-pyrazolidin-3,5-dion,
1-(4-Iod-phenyl)-4-chinolin-4-ylmethylen-pyrazolidin-3,5-dion,
4-[3-(2-Hydroxy-ethoxy)-benzyliden]-1-(4-iod-phenyl)-pyrazolidin-3,5-dion,
1-(4-Iod-phenyl)-4-(5-naphthalin-2-yl-thiophen-2-ylmethylen)-pyrazolidin-3,5-dion,
3-{5-[1-(4-Iod-phenyl)-3,5-dioxo-pyrazolidin-4-ylidenmethyl]-furan-2-yl}thiophen-2-carbonsäuremethylester,
1-(4-lod-phenyl)-4-[5-(2-nitro-4-trifluormethyl-phenyl)-furan-2-ylmethylen]-pyrazolidin-3,5-dion,
4-(5-Chlor-thiophen-2-ylmethylen)-1-(4-iod-phenyl)-pyrazolidin-3,5-dion,
1-(4-lod-phenyl)-4-thiazol-2-ylmethylen-pyrazolidin-3,5-dion,
1-(4-lod-phenyl)-4-(1-methyl-1H-imidazol-2-ylmethylen)-pyrazolidin-3,5-dion,
4-(2-Diethylamino-thiazol-5-ylmethylen)-1-(4-iod-phenyl)-pyrazolidin-3,5-dion,
4-[2-(4-Benzyl-piperazin-1-yl)-th iazol-5-ylmethylen]-1-(4-iod-phenyl)-pyrazolidin-3,5-dion,
1-(4-lod-phenyl)-4-[2-(4-methoxy-phenoxy)-thiazol-5-ylmethylen]-pyrazolidin-3,5-dion,
4-(9-Ethyl-9H-carbazol-3-ylmethylen)-1-(4-iod-phenyl)-pyrazolidin-3,5-dion,
4-Benzo[b]thiophen-3-ylmethylen-1-(4-iod-phenyl)-pyrazolidin-3,5-dion.

9. Alkylidenpyrazolidindion-Derivat, ausgewählt aus der Gruppe, bestehend aus:
4-(2-Hydroxy-benzyliden)-1-(4-iod-phenyl)-pyrazolidin-3,5-dion,
4-[1-(4-Iod-phenyl)-3,5-dioxo-pyrazolidin-4-ylidenmethyl]-benzoesäure,
{4-[1-(4-Iod-phenyl)-3,5-dioxo-pyrazolidin-4-ylidenmethyl]-phenoxy}-essigsäure,
(E)-3-{4-[1-(4-Iod-phenyl)-3,5-dioxo-pyrazolidin-4-ylidenmethyl-phenyl}-acrylsäure,
2-Hydroxy-5-[1-(4-iod-phenyl)-3,5-dioxo-pyrazolidin-4-ylidenmethyl]-benzoesäure,
3-[1-(4-lod-phenyl)-3,5-dioxo-pyrazolidin-4-ylidenmethyl]-benzoesäure,
(E)-3-{4-[3,5-Dioxo-1-(3-trifluoromethyl-phenyl)-pyrazolidin-4-ylidenmethyl]phenyl}-acrylsäure,
{2-[1-(4-lod-phenyl)-3,5-dioxo-pyrazolidin-4-ylidenmethyl]-phenoxy}-essigsäure,
{4-[3,5-Dioxo-1-(3-trifluoromethyl-phenyl)-pyrazolidin-4-ylidenmethyl]-phenoxy}essigsäure,
5-[3,5-Dioxo-1-(3-trifluoromethyl-phenyl)-pyrazolidin-4-ylidenmethyl]-2-hydroxybenzoesäure,
{2-[3,5-Dioxo-1-(3-trifluoromethyl-phenyl)-pyrazolidin-4-ylidenmethyl]-phenoxy}essigsäure,
4-[4-(3-Dimethylamino-propylamino)-benzyliden]-1-(4-iod-phenyl)-pyrazolidin-3,5-dion,
1-(4-lod-phenyl)-4-(4-pyrrolidin-1-yl-benzyliden)-pyrazolidin-3,5-dion,
1-(4-Iod-phenyl)-4-[2-(4-methylpiperazin-1-yl)-benzyliden]-pyxazolidin-3,5-dion,
1-(4-Iod-phenyl)-4-(4-pyridin-2-yl-benzyliden)-pyrazolidin-3,5-dion,
4-[4-(3-Dimethylamino-propoxy)-benzyliden]-1-(4-iod-phenyl)-pyrazolidin-3,5-dion,
4-(Diethylamino-hydroxy-benzyliden)-1-(4-iod-phenyl)-pyrazolidin-3,5-dion.

10. Alkylidenpyrazolidindion-Derivat nach einem der Ansprüche 8 bis 9 zur Verwendung als ein Medikament.

11. Pharmazeutische Zusammensetzung, enthaltend mindestens ein Alkylidenpyrazolidindion-Derivat nach einem der Ansprüche 8 bis 9 und einen pharmazeutisch verträglichen Träger, ein pharmazeutisch verträgliches Verdünnungsmittel oder einen pharmazeutisch verträglichen Arzneimittelträger dafür.

12. Verfahren zum Herstellen von Verbindungen der Formel (III) 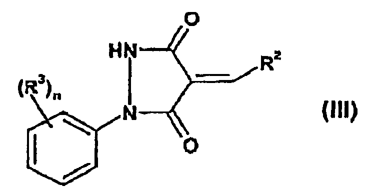 umfassend den Schritt des Umsetzens des Vorläufers (IV) mit dem Aldehyd (V)

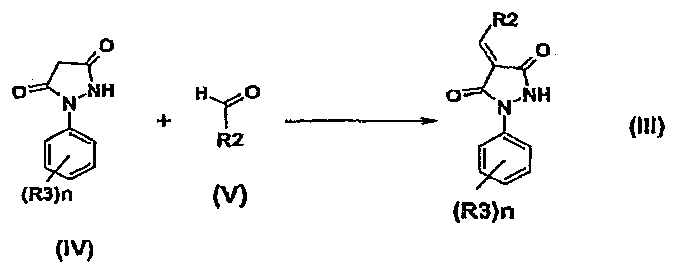

## Revendications

1. Emploi d'un dérivé de type alkylidène pyrazolidine-dione, de formule (I) : 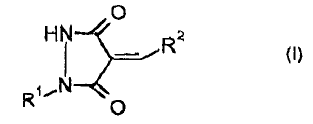 dans laquelle R¹ représente un groupe aryle et R² représente un groupe aryle ou hétéroaryle,
ou de l'un de ses tantomères ou isomères géométriques, ou de l'une de ses formes optiquement actives telles que ses énantiomères, diastéréoisomères et formes racémiques, ou encore de l'un de ses sels pharmacologiquement admissibles, en vue de la préparation d'une composition pharmaceutique conçue pour le traitement et/ou la prévention de troubles métaboliques provoqués par une insulinorésistance ou une hyperglycémie, à savoir le diabète de type I ou II, l'obésité et le syndrome des ovaires polykystiques (SOPK).

2. Emploi conforme à la revendication 1, en vue du traitement et/ou de la prévention du diabète de type II.

3. Emploi conforme à la revendication 1 ou 2, dans lequel R² représente un groupe furanyle, thiényle, pyrrolyle, indolyle, quinolyle ou carbazolyle, qui peut en option porter un substituant alkyle en C₁₋₆, alcényle en C₂₋₆, alcynylc en C₂₋₆, aryle ou hétéroaryle, cycloalkyle ou hétérocycloalkyle de 3 à 8 chaînons, amino, sulfonyle, sulfanyle, sulfinyle, (alkyle en C₁₋₆)sulfonyle, (alkyle en C₁₋₆)sulfinyle, (alkyle en C₁₋₆)sulfanyle, alcoxy, halogéno, y compris iodo, cyano, carboxy, alcoxycarbonyle ou nitro.

4. Emploi conforme à la revendication 3, dans lequel R² représente un groupe furanyle, thiényle, pyrrolyle, indolyle, quinolyle ou carbazolyle, qui peut en option porter un substituant halogéno, carboxy, alcoxycarbonyle
ou nitro.

5. Emploi conforme à l'une des revendications précédentes, dans lequel R² représente un groupe furanyle avec ou sans substituant.

6. Emploi conforme à l'une des revendications précédentes, dans lequel R¹ représente un groupe phényle portant un substituant.

7. Emploi conforme à la revendication 6, dans lequel R¹ représente un groupe iodophényle, trifluorométhylphényle, chlorométhylphényle, méthoxycarbonyl-iodophényle ou biphényle.

8. Dérivé de type alkylidène-pyrazolidine-dione, choisi parmi les suivants ;
- 4-(4-iodobenzylidéne)-1-(4-iodophényl)-pyrazolidine-3,5-dione
- 4-(diméthylfuran-2-yl-méthylène)-1-(3-trifluorométhylphényl)-pyrazolidine-3,5-dione
- 1-(biphényl-4-yl)-4-(furan-2-yl-méthyléne)-pyrazolidine-3,5-dione
- acétate de 5-[1-(4-iodophényl)-3,5-dioxopyrazolidin-4-ylidène-méthyl]-furan-2-yl-méthyle
- 4-(4-bromofuran-2-yl-méthylène)-1-(4-iodophényl)-pyrazolidine-3,5-dione
- 1-(4-iodophényl)-4-(5-méthylfuran-2-yl-méthylène)-pyrazolidine-3,5-dione
- 4-(5-bromofuran-2-yl-méthylène)-1-(4-iodophényl)-pyrazolidine-3,5-dione
- 1-(4-iodophényl)-4-[5-(2-méthoxyphényl)thiophén-2-yl-méthylène]-pyrazolidine-3,5-dione
- 1-(4-iodophényl)-4-(thiophén-3-yl-méthylène)-pyrazolidine-3,5-dione
- 1-(4-iodophényl)-4-(5-phénylthiophén-2-yl-méthylène)-pyrazolidine-3,5-dione
- 4-(furan-2-yl-méthylène)-1-(3-trifluorométhylphényl)-pyrazolidine-3,5-dione
- 4-(5-méthylfuran-2-yl-méthylène)-1-(3-trifluorométhylphényl)-pyrazolidine-3,5-dione
- 4-(thiophén-2-yl-méthylène)-1-(3-trifluorométhylphényl)-pyrazolidine-3,5-dione
- 4-(4-bromofuran-2-yl-méthylène)-1-(3-trifluorométhylphényl)-pyrazolidine-3,5-dione
- acétate de 5-[3,5-dioxo-1-(3-trifluorométhylphényl)-pyrazolidin-4-ylidène-méthyl]-furan-2-yl-méthyle
- 1-(3-chloro-4-méthylphényl)-4-(furan-2-yl-méthylène)-pyrazolidine-3,5-dione
- 4-(7-bromo-8-hydroxyquinol-5-yl-méthylène)-1-(4-iodophényl)-pyrazolidine-3,5-dione
- 1-(4-iodophényl)-4-[5-(4-nitrophényl)-furan-2-yl-méthylène]-pyrazolidine-3,5-dione
- 1-(4-iodophényl)-4-[5-(2-nitrophényl)-furan-2-yl-méthylène]-pyrazolidine-3,5-dione
- 4-(5-hydroxyméthyl-furan-2-yl-méthylène)-1-(4-iodophényl)-pyrazolidine-3,5-dione
- 4-((E)-3-furan-2-yl-allylidène)-1-(4-iodophényl)-pyrazolidilne-3,5-dione
- 4-(furan-3-yl-méthylène)-1-(4-iodophényl)-pyrazolidine-3,5-dione
- 1-(4-iodophényl)-4-(5-nitrothiophén-2-yl-méthylène)-pyrazolidine-3,5-dione
- 4-(5-hydroxyméthylfuran-2-yl-méthylène)-1-(3-trifluorométhylphényl)-pyxazolidine-3,5-dione
- 4-(furan-3-yl-méthylèue)-1-(3-trifluorométhylphényl)-pyrazolidine-3,5-dione
- 4-(5-nitrothiophén-2-yl-méthylène)-1-(3-trifluorométhylphényl)-pyrazolidine-3,5-dione
- 1-(4-iodophényl)-4-(5-nitrofuran-2-yl-méthylène)-pyrazolidine-3,5-dione
- 1-(4-iodophényl)-4-[5-(3-nitrophényl)furan-2-yl-méthylène]-pyrazolidine-3,5-dionc
- 4-(4,5-diméthylfuran-2-yl-méthylène)-1-(4-iodophényl)-pyrazolidine-3,5-dione
- acide 5-[1-(4-iodophényl)-3,5-dioXopytazolidin-4-ylidène-méthyl]-furanc-2-sulfonique
- 4-[1-(4-iodophényl)-3,5-dioxopyrazolidin-4-ylidène-methyl]benzonitrile
- 1-(4-iodophényl)-4-(3-méthylthiophén-2-yl-méthylène)-pyrazolidine-3,5-dione
- 4-(4-bromothiophén-2-yl-méthylène)-1-(4-iodophényl)-pyrazolidine-3,5-dione
- 1-(4-iodophényl)-4-(5-méthylthiophén-2-yl-méthylène)-pyrazolidine-3,5-dione
- 4-(benzo[b]thiophén-2-yl-méthylène)-1-(4-iodophényl)-pyrazolidine-3,5-dione
- 1-(4-iodophényl)-4-(4-nitrothiophén-2-yl-méthylène)-pyrazolidine-3,5-dione
- acide 5-[1-(4-iodophényl)-3,5-dioxopyrazolidin-4-ylidène-méthyl]-thiophène-3-carboxylique
- acide 4-[1-(4-iodophényl)-3,5-dioxopyxazolidin-4-ylidène-méthyl]-thiophène-3-carboxylique
- acide 2-[3,5-dioxo-1-(3-trifluorométhylphenyl)-pyrazolidin-4-ylidène-méthyl]-thiophène-3-carboxylique
- 4-(4-nitrothiophén-2-yl-méthyléne)-1-(3-trifluorométhylphényl)-pyrazolidine-3,5-dione
- acide 4-[3,5-dioxo-1-(3-trifluotométhylphényl)-pyrazolidin-4-ylidène-méthyl]-thiophène-3-carboxylique
- acide {3-[3,5-dioxo-1-(3-trifluorométhylphényl)-pyrazolidin-4-ylidène-méthyl]-thiophén-2-yl-sulfanyl}-acétique
- acide 2-[1-(4-iodophényl)-3,5-dioxopyrazolidin-4-ylidène-méthyl]-thiophène-3-carboxylique
- acide {3-[1-(4-iodophényl)-3,5-dioxopyrazolidin-4-ylidène-méthyl]-thiophén-2-yl-sulfanyl}-acétique
- 4-(4-hydroxyméthylfuran-3-yl-méthylène)-1-(3-trifluorométhylphényl)-pyrazolidine-3,5-dione
- 4-(benzo[b]thiophén-2-yl-méthylène)-1-(3-trifluorométhylphényl)-pyrazolidine-3,5-dione
- acide 5-[3,5-dioxo-1-(3-trifluorométhylphényl)-pyrazolidin-4-ylidène-méthyl]-thiophène-3-carboxylique
- acide 5-[1-(4-iodophényl)-3,5-dioxopyrazolidin-4-ylidène-méthyl]-furanc-2-carboxylique
- acide 5-[1-(biphényl-4-yl)-3,5-dioxopyrazolidin-4-ylidène-méthyl]-furane-2-carboxylique
- 4-(4-hydroxynapht-1-yl-méthylène)-1-(4-iodophényl)-pyrazolidine-3,5-dione
- 1-(4-iodophényl)-4-(1-oxypyrid-4-yl-méthylène)-pyrazolidine-3,5-dione
- 2-[4-(furan-2-yl-méthylène)-3,5-dioxopyrazolidin-1-yl]-5-iodobenzoate de méthyle
- 4-(1H-indol-5-yl-méthylène)-1-(4-iodophényl)-pyrazolidine-3,5-dione
- 4-(4-hydroxyméthylfuran-3-yl-méthylène)-1-(4-iodophényl)-pyrazolidine-3,5-dione
- 1-(4-iodophényl)-4-(1H-pyrrol-2-yl-méthylène)-pyrazolidine-3,5-dione
- 1-(4-iodophényl)-4-(1-méthyl-1H-pyrrol-2-yl-méthylène)-pyrazolidine-3,5-dione
- 1-(4-iodophényl)-4-[1-(2-nitrobenzyl)-1H-pyrrol-2-yl-méthylène]-pyrazolidine-3,5-dione
- 1-(4-iodophényl)-4-(1H-pyrazol-3-yl-méthylène)-pyrazolidine-3,5-dione
- 1-(4-iodophényl)-4-(1H-pyrazol-3-yl-méthylène)-pyrazolidine-3,5-dione
- 4-(2,3-dihydrobenzofuran-5-yl-méthylène)-1-(4-iodophényl)-pyrazolidine-3,5-dione
- 1-(4-iodophényl)-4-(quinol-2-yl-méthylène)-pyrazolidine-3,5-dione
- 1-(4-iodophényl)-4-(quinol-4-yl-méthylène)-pyrazolidine-3,5-dione
- 4-[3-(2-hydroxyéthoxy)benzylidène]-1-(4-iodophényl)-pyrazolidine-3,5-dione
- 1-(4-iodopbényl)-4-[5-(napht-2-yl)-thiophén-2-yl-méthylène]-pyrazolidine-3,5-dione
- 3-{5-[1-(4-iodophényl)-3,5-dioxopyrazolidin-4-ylidène-méthyl]-furan-2-yl}-thiophène-2-carboxylate de méthyle
- 1-(4-iodophényl)-4-[5-(2-nitro-4-trifluorométhylphényl)-furan-2-yl-méthylène]-pyrazolidine-3,5-dione
- 4-(5-chlorothiophén-2-yl-méthyléne)-1-(4-iodophényl)-pyrazolidine-3,5-dione
- 1-(4-iodophényl)-4-(thiazol-2-yl-méthylène)-pyrazolidine-3,5-dione
- 1-(4-iodophényl)-4-(1-methyl-1H-imidazol-2-yl-méthylène)-pyrazolidine-3,5-dionc
- 4-(2-diéthylaminothiazol-5-YI-méthylène)-1-(4-iodophényl)-pyrazolidinc-3,5-dionc
- 4-[2-(4-benzyl-pipérazin-1-yl)-thiazol-5-yl-méthylène]-1-(4-iodophényl)-pyrazolidine-3,5-dione
- 1-(4-iodophényl)-4-[2-(4-méthoxyphénoxy)-thiazol-5-yl-méthylène]-pyrazolidine-3,5-dione
- 4-(9-éthyl-9H-carbazol-3-yl-méthylène)-1-(4-iodophényl)-pyrazolidine-3,5-dione
- 4-(benzo[b]thiophén-3-yl-méthylène)-1-(4-iodophényl)-pyrazolidine-3,5-dione

9. Dérivé de type alkylidène-pyrazolidine-dione, choisi parmi les suivants :
- 4-(2-hydroxybenzylidène)-1-(4-iodophényl)-pyrazolidine-3,5-dione
- acide 4-[1-(4-iodophényl)-3,5-dioxopyrazolidin-4-ylidène-méthyl]-benzoïque
- acide {4-[1-(4-iodophényl)-3,5-dioxopyrazolidin-4-ylidène-méthyl]-phénoxy}-acétique
- acide (E)-3-{4-[1-(4-iodophényl)-3,5-dioxopyrazolidin-4-ylidène-méthyl]-phényl}-acrylique
- acide 2-hydroxy-5-[1-(4-iodophényl)-3,5-dioxopyrazolidin-4-ylidène-méthyl]-benzoïque
- acide 3-[1-(4-iodophényl)-3,5-dioxopyrazolidiD-4-ylidène-méthyl]-benzoïque
- acide (E)-3-(4-[3,5-dioxo-1-(3-trifluorométhylphényl)-pyrazolidin-4-ylidène-méthyl]-phényl)-acrylique
- acide {2-[1-(4-iodophényl)-3,5-dioxopyrazolidin-4-ylidène-méthyl]-phénoxy}-acétique
- acide {4-[3,5-dioxo-1-(3-trifluorométhylphényl)-pyrazolidin-4-ylidène-méthyl]-phénoxy}-acétique
- acide 5-[3,5-dioxo-1-(3-trifluorométhylphényl)-pyrazolidin-4-yldène-méthyl]-2-hydroxy-benzoïque (10)
- acide {2-[3,5-dioxo-1-(3-trifluorométhylphényl)-pyrazolidin-4-ylidène-méthyl]-phénoxy }-acétique
- 4-[4-(3-diméthylaminopropylamino)-benzylidène]-1-(4-iodophényl)-pyrazolidine-3,5-dione
- 1-(4-iodophényl)-4-(4-pyrrolidin-1-yl-benzylidène)-pyrazolidine-3,5-dione
- 1-(4-iodophényl)-4-[2-(4-méthylpipérazin-1-yl-benzylidène)-pyrazolidine-3,5-dione
- 1-(4-iodophényl)-4-(4-pyrid-2-yl-benzylidène)-pyrazolidine-3,5-dione
- 4-[4-(3-diméthylaminopropoxy)-benzylidéne]-1-(4-iodophényl)-pyrazolidine-3,5-dione
- 4-(diéthylamino-hydroxy-benzylidène)-1-(4-iodophényl)-pyrazolidine-3,5-dione.

10. Dérivé de type alkylidène-pyrazolidine-dione, conforme à l'une des revendications 8 et 9, destiné à être employé comme médicament.

11. Composition pharmaceutique contenant au moins un dérivé de type alkylidène-pyrazolidino-dione, conforme à l'une des revendications 8 et 9, ainsi qu'un véhicule, diluant ou excipient pharmacologiquement admissible pour ce dérivé,

12. Procédé de préparation de composés de formule (III) : 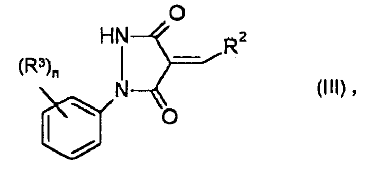 lequel procédé comporte une étape qui consiste à faire réagir un précurseur (IV) avec un aldéhyde (V) :
